# EUROPEAN PATENT SPECIFICATION

(11) **EP 1 887 297 B1**
(45) Date of publication and mention of the grant of the patent: **26.04.2017**
(21) Application number: 06756551.5
(22) Date of filing: 24.05.2006
(51) Int. Cl.: F25D 11/02, F25D 19/00, F25D 23/00, F25D 17/04, A61L 2/10

(54) **REFRIGERATOR**
KÜHLSCHRANK
RÉFRIGÉRATEUR

(30) Priority: 26.05.2005 JP 2005153498; 26.05.2005 JP 2005153501; 22.06.2005 JP 2005181966; 31.10.2005 JP 2005316135; 18.04.2006 JP 2006114195
(43) Date of publication of application: 13.02.2008
(73) Proprietor: Panasonic Corporation, Kadoma-shi Osaka 571-8501 (JP)
(72) Inventor: KOJIMA, Yoshiko,Matsushita Electric Ind. Co., Ltd., Shiromi,Chuo-ku,Osaka-shi,Osaka 540-6207 (JP); ASHIDA, Yae,Matsushita Electric Ind. Co., Ltd., Shiromi,Chuo-ku,Osaka-shi,Osaka 540-6207 (JP); HIROTA, Yukiko,Matsushita Electric Ind. Co., Ltd., Shiromi,Chuo-ku,Osaka-shi,Osaka 540-6207 (JP); ISHITA, Mitoko,Matsushita Electric Ind. Co., Ltd., Shiromi,Chuo-ku,Osaka-shi,Osaka 540-6207 (JP)
(74) Representative: Pautex Schneider, Nicole Véronique
(86) International application number: PCT/JP2006/310345
(87) International publication number: WO 2006/126584

(56) References cited:
- JP-A- 2002 350 039
- JP-A- 2003 287 352
- JP-A- 2003 287 357
- JP-A- 2003 322 460
- JP-A- 2005 055 031

## Description

### TECHNICAL FIELD

The invention relates to a refrigerator in which an LED emits ultraviolet rays to a storage compartment to improve the shelf life of articles.

### BACKGROUND ART

With an increase in the entry of women in public affairs, many women prefer to use a method of storing food in a temperature range close to the freezing point, such as a partial freezing temperature range, since thawing is not needed unlike a freezing temperature range, rapid cooking is possible, and the freshness of food is safely maintained. However, it is difficult to completely prevent the proliferation of microorganisms or mold in these temperature ranges. When food is stored with various bacteria deposited thereto, the inside of the storage compartment is full of a foul odor, the microorganisms or mold are spread to other stored articles, or food may be decayed, which accelerates the proliferation of mold.

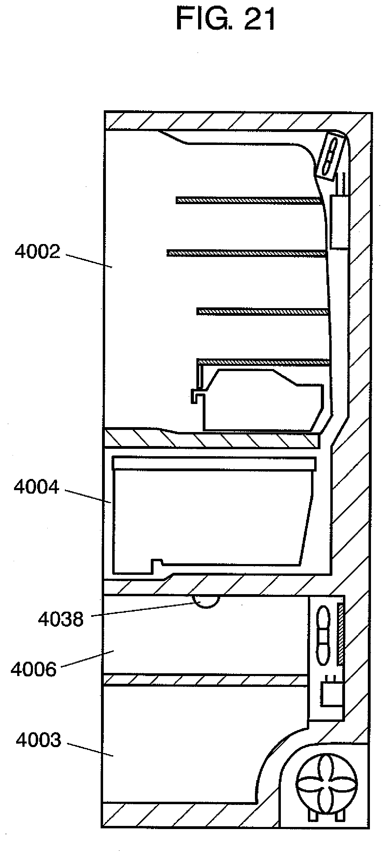

In order to solve these problems, as shown in Fig. 21, a refrigerator disclosed in Japanese Patent Unexamined Publication No. 2003-287357 includes refrigerating compartment 4002, vegetable compartment 4004, temperature switching chamber 4006, and freezing compartment 4003. In addition, a drawer-type case is provided in temperature switching chamber 4006, and ultraviolet lamp 4038 is provided on the ceiling of temperature switching chamber 4006. Ultraviolet rays emitted from ultraviolet lamp 4038 remove various microorganisms deposited on the surface of food stored in the case. That is, in the refrigerator according to the related art, an antimicrobial performance is improved by using ultraviolet lamp 4038 provided on the ceiling of temperature switching chamber 4006.

However, in the refrigerator according to the related art, since temperature switching chamber 4006 is composed of a drawer-type case, meat or fish stored in temperature switching chamber 4006 comes into contact with the air outside the refrigerator when the door is opened, which may causes the decay of food due to the infiltration of various microorganisms. In addition, when the door is opened, the internal temperature of the chamber increases, which may cause the quality of food to deteriorate. The ultraviolet rays having a short wavelength emitted from ultraviolet lamp 4038 provided on the ceiling of switching chamber 4006 may accelerate the deterioration of resin or the lipid oxidation of food.

JP 2003287352 and JP 2005-055031 discloses a refrigerator according to the preamble of claim 1.

### DISCLOSURE OF THE INVENTION

According to the invention, a refrigerator is as defined in claim 1.

### BRIEF DESCRIPTION OF THE DRAWINGS

Fig. 1 is a side cross-sectional view illustrating a refrigerator according to a first example not forming part of the invention.
Fig. 2 is a side cross-sectional view illustrating a refrigerator according to a second example not forming part of the invention.
Fig. 3 is a side cross-sectional view illustrating a refrigerator according to the invention.
Fig. 4 is a cross-sectional view illustrating a light source of the refrigerator according to the invention.
Fig. 5 is a diagram illustrating the relationship between the amount of light emitted from an ultraviolet LED and the number of microorganisms when a salmon is stored for one week in the refrigerator according to the invention.
Fig. 6 is a side cross-sectional view illustrating a refrigerator according to a third example not forming part of the invention.
Fig. 7 is a side cross-sectional view illustrating a refrigerator according to a fourth example not forming part of the invention.
Fig. 8 is a side cross-sectional view illustrating a refrigerator according to a fifth example not forming part of the invention.
Fig. 9 is a side cross-sectional view illustrating a refrigerator according to a sixth example not forming part of the invention.
Fig. 10 is a cross-sectional view illustrating a light source of the refrigerator according to the sixth example not forming part of the invention.
Fig. 11 is a front view illustrating a refrigerator according to a seventh example not forming part of the invention.
Fig. 12 is a front view illustrating the refrigerator according to the seventh example not forming part of the invention with doors being removed.
Fig. 13 is a partial enlarged front view illustrating a storage case according to seventh example not forming part of the invention.
Fig. 14 is a partial enlarged front view illustrating the storage case according to seventh example not forming part of the invention.
Fig. 15 is a diagram illustrating the relationship between the amount of light emitted from an ultraviolet LED and the number of microorganisms when a salmon is stored for one week in the refrigerator according to the seventh example not forming part of the invention.
Fig. 16 is a side cross-sectional view illustrating a refrigerator according to an eighth example not forming part of the invention.
Fig. 17 is a diagram illustrating an agricultural chemical removing performance of ultraviolet rays in the refrigerator according to the eighth example not forming part of the invention.
Fig. 18 is a side cross-sectional view illustrating a refrigerator according to a ninth example not forming part of the invention.
Fig. 19 is a diagram illustrating an agricultural chemical removing performance of ultraviolet rays in the refrigerator according to the ninth example not forming part of the invention.
Fig. 20 is a side cross-sectional view illustrating a refrigerator according to a tenth example not forming part of the invention.
Fig. 21 is a side cross-sectional view illustrating a refrigerator according to the related art.

### REFERENCE NUMERALS

1, 201, 301, 2001, 2101: REFRIGERATOR BODY
2, 202, 302, 2002, 3112: REFRIGERATING COMPARTMENT
3, 203, 303, 2003, 3115: FREEZING COMPARTMENT
4, 2004, 3114: VEGETABLE COMPARTMENT
6, 206, 306, 2006, 3113: SWITCHING CHAMBER (STORAGE COMPARTMENT)
12, 212, 312, 2012: MACHINE ROOM
13, 213, 313, 2013: LIGHT SOURCE
16, 1216, 1316, 2016: COMPRESSOR
20, 2020: EVAPORATOR
35, 2035: STORAGE CASE (PARTITIONED REGION)
3100: REFRIGERATOR
3111: PARTITION WALL
3116a, 3116b: DOOR
3118: ULTRAVIOLET LAMP (ULTRAVIOLET EMITTING UNIT)
3120: DOOR DETECTOR
3121: OPERATING SWITCH

### PREFERRED EMBODIMENTS FOR CARRYING OUT THE INVENTION

Hereinafter, preferred embodiment of the invention and examples not forming part of the invention will be described below with reference to the accompanying drawings. However, the invention is not limited to the embodiment.

### (First example not forming part of the invention)

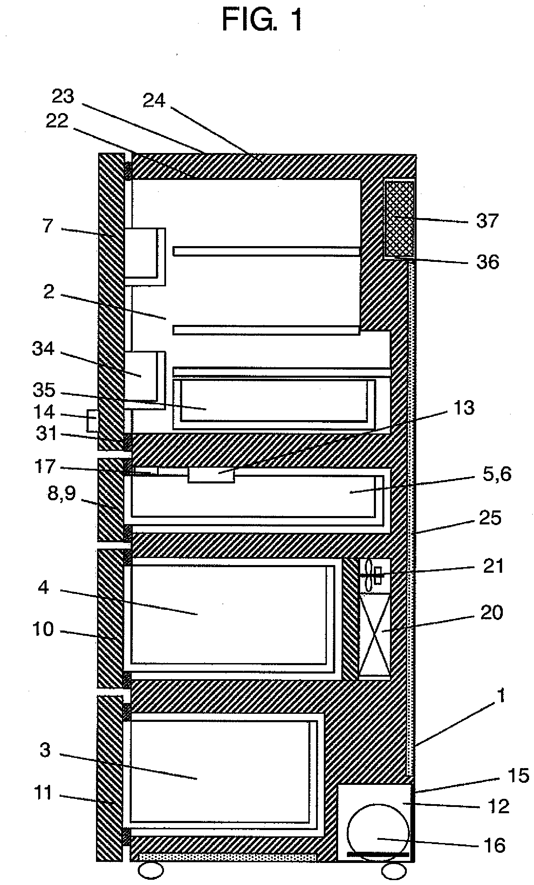

Fig. 1 is a side cross-sectional view illustrating a refrigerator according to a first example not forming part of the invention.

In Fig. 1, refrigerator body (hereinafter, simply referred to as body) 1 includes inner case 22 that is formed of resin, such as ABS, by vacuum forming, outer case 23 that is formed of a metal material, such as a pre-coat steel sheet, and an insulating wall composed of foam insulation 24 that is provided between inner case 22 and outer case 23. For example, rigid urethane foam, phenol foam, or styrene foam may be used as foam insulation 24. A hydrocarbon-based cyclopentane may be used as a foam material to prevent global warming.

Vacuum insulating material 25 is closely adhered to the outer case by an adhesive (not shown) in a space between inner case 22 and outer case 23 before foam insulation 24 is foamed. Vacuum insulating material 25 is formed of a thin plane since it is provided inside the wall of body 1. In addition, an adhesive, such as a hot-melt adhesive, is coated on the entire surface of vacuum insulating material 25 to prevent air from being infiltrated into the adhered portion. Vacuum insulating material 25 is integrally foamed with foam insulation 24 to form body 1. Therefore, vacuum insulating material 25 has an insulating performance that is 5 to 20 times higher than that of foam insulation 24 to improve the insulating performance of the insulating wall.

Body 1 is divided into a plurality of insulating sections, and storage compartments are provided in the divided sections. A hinged door is provided in an upper compartment, and a drawer-type door is provided in a lower compartment. Specifically, body 1 is partitioned into refrigerating compartment 2, drawer-type switching chamber 6 and drawer-type ice making compartment 5 that are provided in parallel to each other, drawer-type vegetable compartment 4, and drawer-type freezing compartment 3 in this order from the upper side. The divided insulating sections are provided with insulating doors with gasket 31 interposed therebetween. In addition, body 1 includes hinged door 7 of the refrigerating compartment, drawer-type door 8 of the switching chamber, drawer-type door 9 of the ice making compartment, drawer-type door 10 of the vegetable compartment, and drawer-type door 11 of the freezing compartment in this order from the upper side.

Hinged door 7 of the refrigerating compartment is provided with door pocket 34, which serves as a storage space, and a plurality of shelves are provided in the refrigerating compartment. Further, storage case 35 is provided at the lowermost part of refrigerating compartment 2.

The temperature of refrigerating compartment 2 is generally set in the range of 1 to 5°C, which is a lower limit that is capable of storing food or other substances without freezing them. However, a user can arbitrarily set the temperature of refrigerating compartment 2 according to the kind of articles to be stored. In addition, the temperature of refrigerating compartment 2 may be set to a relatively high temperature of about 10°C in order to preserve wine or vegetables with roots.

The internal temperature of storage case 35 is set to a relatively low temperature of -3 to 1°C in order to improve the degree of freshness of fishes, fish-processed goods, and milk products. Vegetable compartment 4 is generally set to a temperature that is equal to or slightly higher than that of refrigerating compartment 2, that is, in a temperature range of 2 to 7°C. As the internal temperature of vegetable compartment 4 becomes lower, it possible to maintain the degree of freshness of vegetables for a longer time.

The user can change the internal temperature of switching chamber 6. The internal temperature of switching chamber 6 can be changed within a predetermined temperature range from the temperature of the freezing compartment to a partial freezing temperature range and a refrigerating temperature range. The user can operate switch 14 provided on hinged door 7 of the refrigerating compartment to adjust the internal temperature of switching chamber 6. The internal temperature of switching chamber 6 is detected by detector 17. In this way, switching chamber 6, as a thermally insulated storage compartment ,has a function for controlling the storage compartment to one of a plurality of predetermined temperatures. Further, light source 13 is provided in switching chamber 6. That is, the refrigerator according to this example includes the thermally insulated storage compartment, and an LED, which serves as light source 13 having a wavelength of a ultraviolet region, in the storage compartment. The ultraviolet ray emitted from the LED, serving as light source 13, has a wavelength range of 280 nm to 400 nm. The ultraviolet ray emitted form the light source prevents the proliferation of microorganisms deposited on the surface of food or the inner surface of the switching chamber in the refrigerator, and thus the shelf life of fishes is improved. In addition, an LED that emits visible light having a wavelength range of 400 nm to 800 nm may be provided in addition to the LED emitting ultraviolet rays, serving as light source 13. In this case, when the drawer-type door is pulled out, visible light emitted from light source 13 leaks to the outside, which makes it possible for the user to view light emitted from the refrigerator. The number of light sources 13 or the amount of light emitted from the light source is not particularly limited. The light source may emit an optimum color light component at an optimum intensity, if necessary, and the on or off state of the light source is controlled by control substrate 37.

Ice making compartment 5 is an independent ice storage box. Ice making compartment 5 has an automatic ice maker (not shown) to automatically make ice, and stores ice. The internal temperature of ice making compartment 5 is set to a freezing temperature range to store ice. However, the internal temperature of ice making compartment 5 can be set in a temperature range that is slightly higher than the freezing temperature range since its main object is to store ice.

The internal temperature of freezing compartment 3 is generally set in the range of -22 to -18°C in order to freeze food. However, the internal temperature of freezing compartment 3 can be set in the range of -30 to -25°C in order to keep refrigerated food in a better state.

Body 1 has machine room 12 at a rear lower side. In addition, body 1 has second machine room 36 at the upper side of machine room 12.

A refrigeration cycle is composed of compressor 16 provided in machine room 12, a condenser (not shown), a capillary, serving as a decompression device, and evaporator 20, which are connected to each other in a ring shape. The forced convection thermal exchange of evaporator 20 is performed by cooling fan 21. The condenser (not shown) may be cooled down by a forced air cooling method using a fan, or it may be cooled down by a natural air cooling method by providing the condenser inside outer case 23 such that heat is transmitted. Alternatively, the condenser may be provided in a partition between insulating doors and connected to a pipe for preventing water droplets.

A plurality of evaporators may be switched according to a partitioned structure or a set temperature, by a water-course controller, such as a motor-driven three-way valve, or a plurality of capillaries may be switched. When compressor 16 is in an off state, the circulation of cooling gas may stop.

Control substrate 37 for operating the refrigeration cycle is provided in second machine room 36 so as to be sealed by a detachable cover. Machine room 12 is substantially sealed by detachable rear cover 15.

Evaporator 20, which is a component of the refrigeration cycle, and cooling fan 21 are provided at the rear side of vegetable compartment 4 in the meddle stage. This structure makes it possible to maximize the volume and depth of freezing compartment 3, which is a storage compartment provided at the lowest stage.

Vegetable compartment 4 in the middle stage and freezing compartment 3 in the lowest stage may be reversed. In this case, it is possible to maximize the volume and depth of vegetable compartment 4.

The operation and effect of the refrigerator having the above-mentioned structure will be described below.

First, the operation of the refrigeration cycle will be described below. The refrigeration cycle is operated in response to signals output from control substrate 37 according to a set temperature to perform a cooling operation. A high-temperature and high-pressure cooling medium discharged by the operation of compressor 16 is condensed into liquid by the condenser, and the pressure of the condensed liquid is reduced by the capillary, and a low-pressure and low-temperature liquid cooling medium is supplied to evaporator 20.

Thermal exchange occurs between the cooling medium in evaporator 20 and the air in the refrigerator by the operation of cooling fan 21, and the cooling medium is evaporated. A damper (not shown) distributes a low-temperature cooling air to the storage compartments to reduce the internal temperatures thereof. When a plurality of evaporators or decompression devices are used, the water course controller supplies the cooling medium to necessary evaporators 20. The cooling medium discharged from evaporator 20 is supplied to compressor 16. The refrigeration cycle is repeatedly performed to reduce the internal temperature of the refrigerator.

The internal temperature of switching chamber 6 can be changed in several stages from a freezing temperature range to a refrigerating temperature range, according to the user's purpose or favorite. In this example, the internal temperature of switching chamber 6 can be changed to a refrigerating temperature of 3°C, a freezing temperature of - 18°C, a partial freezing temperature of -3 to -2°C, which is an intermediate temperature between the refrigerating temperature and the freezing temperature, and a chilled temperature of -2 to 0°C.

An LED emitting light having a wavelength range of 280 nm to 400 nm, which is the range of relatively safe ultraviolet rays A and B, is used as light source 13, which makes it possible to inactivate the proliferation of microorganisms that are floating in the storage compartment or deposited on the surface of food or the inner wall of the storage compartment by changing genes of the microorganisms. In this way, it is possible to maintain the hygienic conditions in the storage compartment, and thus delay the discoloration, foul odor, and slime on the surface of food caused by microorganisms. Therefore, the refrigerator can improve the shelf life of food using light including ultraviolet rays that is emitted from light source 13, and sanitarily store food.

Further, some kinds of mushrooms and fishes contain a lot of precursors of vitamin D. When an ultraviolet ray is emitted to the precursors, molecules of the precursors are excited, and are then transformed into vitamin D. Therefore, the refrigerator can store food while increasing the content of vitamin D in a specific food stored in switching chamber 6 by providing light source 13 emitting ultraviolet rays in switching chamber 6.

Light source 13 includes an LED emitting light having a wavelength range of 280 nm to 400 nm, which is the range of relatively safe ultraviolet rays A and B, and an LED that emits red light having a wavelength of about 650 nm. Therefore, light emitted from light source 13 can accelerate the generation of anthocyanin, which is a red plant pigment that is generally contained in, for example, apples, blueberries, strawberries, green beefsteak plants, broccoli, eggplants, and purple sweet potatoes. Since anthocyanin is generally contained in vegetables or fruits, switching chamber 6 is set to a temperature suitable for storing vegetables. In addition, it is proved that anthocyanin, which is a kind of polyphenol, is efficacious for eyes, antiaging by antioxidative activities, and the hardening of the arteries. Therefore, it is considered that anthocyanin is good for the health. Thus, the refrigerator according to this example can store vegetables or fruits while increasing the amount of anthocyanin.

Light source 13 includes an LED emitting light having a wavelength range of 280 nm to 400 nm, which is the range of relatively safe ultraviolet rays A and B, and an LED that emits visible light having a wavelength of 400 nm or more. It is possible to emit light that is similar to sunlight by controlling the wavelengths of light emitted from the LEDs. In addition, it is possible to control the LED to emit visible light, such as red, green, and blue, according to a temperature range set in switching chamber 6. A change in the color of light according to the set temperature enables the user to visibly recognize the set temperature of switching chamber 6 or a variation in the temperature of switching chamber. In addition, the user can easy determine what kind of articles is stored in switching chamber 6 on the basis of the color of light. Therefore, it is possible to prevent the confusion of the user when the user stores food in switching chamber 6, and the user can store food suitable for a set temperature in the switching chamber.

Light source 13 is controlled on the basis of the result detected by detector 17. A temperature sensor is used as detector 17, and when detector 17 detects a predetermined temperature, a signal corresponding to the detected result is input to control the light source. For example, when a door switch is used as detector 17, a control signal may be input when a predetermined time has elapsed after the opening of the door is detected, but the invention is not limited thereto.

In the refrigerator according to this example, switching chamber 6 is provided below refrigerating compartment 2 and above vegetable compartment 4 and freezing compartment 3. This layout enables a woman having an average height to open the door of switching chamber 6 and to take out food from switching chamber 6, without bending her back, which makes it possible for the user to conveniently use the refrigerator. Therefore, the usability of the refrigerator is improved, and the arrangement of the light source 13 makes it possible to further improve the shelf life of food.

According to the first example, considering the usability of the refrigerator, a hinged door is used for the refrigerating compartment, and drawer-type doors are used for the other storage compartments. However, the invention is not limited thereto.

In switching chamber 6 of the refrigerator according to this example, antibiosis inside switching chamber 6 is improved by light emitted from light source 13 including an LED emitting light having a wavelength range of 280 nm to 400 nm, which is the range of relatively safe ultraviolet rays A and B, and an LED that emits visible light having a wavelength of 400 nm or more, and a function of increasing the amount of vitamin D in food containing a vitamin D precursor, such as a dried anchovy, is also improved by light emitted from light source 13. As a result, the storage performance of switching chamber 6 is improved.

### (Second example not forming part of the invention)

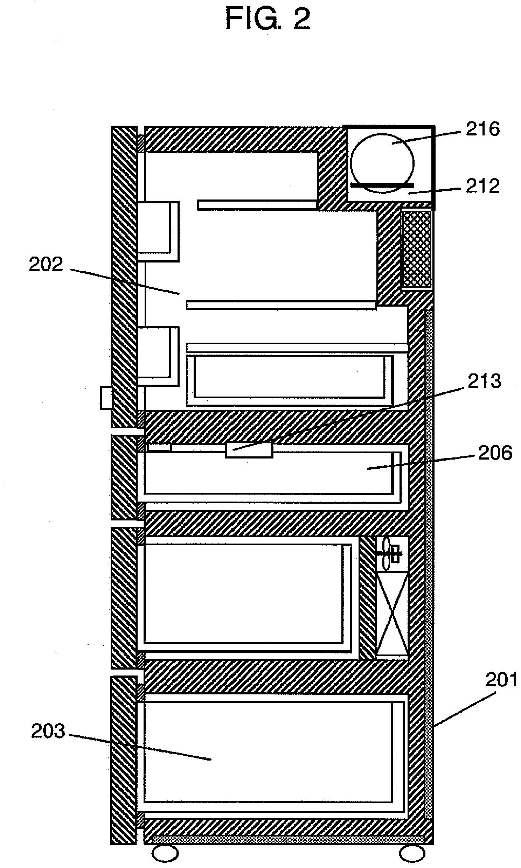

Fig. 2 is a side cross-sectional view illustrating a refrigerator according to a second example not forming part of the invention.

In Fig. 2, light source 213 is provided in switching chamber 206. Machine room 212 housing the main components of a refrigeration cycle, such as compressor 216 and a condenser (not shown), is provided at a rear upper side of refrigerating compartment 202, not at the rear side of freezing compartment 203.

In the related art, since machine room 212 is arranged on the rear side of freezing compartment 203, which is the lowest part of body 201, the machine room reduces the volume of freezing compartment 203. When a freezing space is insufficient in freezing compartment 203, in many cases, the switching chamber is used as the freezing compartment by adjusting the internal temperature thereof, and frozen food is stored in the switching chamber. However, in this example, machine room 212 is provided at the rear upper side of refrigerating compartment 202, which is a place that has not been used in the refrigerator according to the related art since it is too high to touch. Therefore, a space corresponding to the machine room according to the related art can be used as a storage space, which makes it possible to increase the effective volume of freezing compartment 203 without increasing the size of a refrigerator.

In recent years, a bargain sale on frozen food has been frequently conducted in supermarkets, and consumers have a tendency to purchase the frozen food at only a special bargain day. Therefore, a space for storing the frozen food may be insufficient. In the related art, in order to compensate for the insufficient volume of freezing compartment 203, which is the lowest part of body 201, switching chamber 206 is frequently used in a freezing mode. However, in this example, since the volume of freezing compartment 203 increases, switching chamber 206 can be used for various purposes other than freezing.

For example, when switching chamber 206 is set in a partial freezing mode, fishes can be preserved for a week in a partial freezing temperature range of about -3°C. Therefore, the user can cook the fished without thawing. In addition, since partial freezing storage causes less damage to the cells of fishes than freezing storage, less drip is generated from food stored in the partial freezing mode, and the quality of food can be maintained in a better state. Further, antibiosis inside switching chamber 206 is improved by light emitted from light source 213 including an LED emitting light having a wavelength range of 280 nm to 400 nm, which is the range of relatively safe ultraviolet rays A and B, and an LED that emits visible light having a wavelength of 400 nm or more, and a function of increasing the amount of vitamin D in food containing a vitamin D precursor, such as a dried anchovy, is also improved by light emitted from light source 213. As a result, the storage performance of switching chamber 206 is improved.

### (Embodiment of the invention)

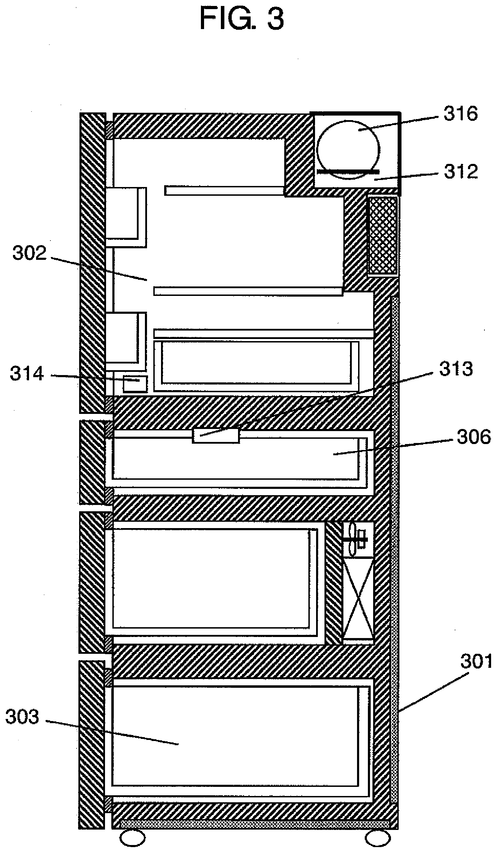

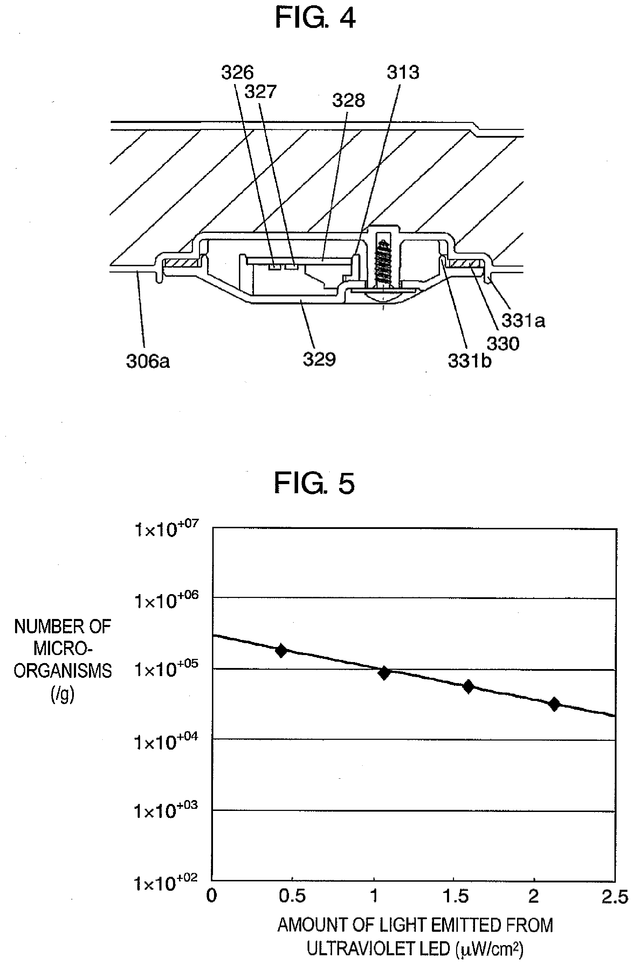

Fig. 3 is a side cross-sectional view illustrating a refrigerator according to an embodiment of the invention. Fig. 4 is a cross-sectional view illustrating a light source provided in a switching chamber according to the embodiment of the invention. Fig. 5 is a graph illustrating the relationship between the amount of ultraviolet rays emitted from an LED and the number of microorganisms when a salmon is stored for one week in the switching chamber according to the embodiment of the invention.

In this embodiment, machine room 312 housing the main components of a refrigeration cycle, such as compressor 316 and a condenser (not shown), is provided at a rear upper side of refrigerating compartment 302, not at the rear side of freezing compartment 303. Therefore, a space corresponding to machine room 312 according to the related art can be used as a storage space, which makes it possible to increase the effective volume of freezing compartment 303 without increasing the size of a refrigerator. In the related art, in order to compensate for the insufficient volume of freezing compartment 303, which is the lowest part of body 301, switching chamber 306 is frequently used in a freezing mode. However, in this embodiment, since the volume of freezing compartment 303 increases, switching chamber 306 can be used for various purposes other than freezing. For example, when switching chamber 306 is set in a partial freezing mode, fishes can be preserved for a week in a partial freezing temperature range of about -3°C. Therefore, the user can cook the fishes without thawing. In addition, since partial freezing storage causes less damage to the cells of fishes than freezing storage, less drip is generated from food stored in the partial freezing mode, and the quality of food can be maintained in a better state.

As shown in Fig. 3, light source 313 is provided on the ceiling of switching chamber 306, and operating panel 314 is provided on the inner wall of refrigerating compartment 302.

In Fig. 4, light source 313 includes light source substrate 328 having ultraviolet LED 326 that emits an ultraviolet region having a peak wavelength of 387 nm and blue LED 327 that emits blue light having a peak wavelength of 468 nm provided thereon, and is covered with transparent cover 329. In addition, light source 313 is arranged at a substantially central portion of ceiling 306a of switching chamber 306.

Ultraviolet LED 326 and blue LED 327, which are light sources provided on light source substrate 328, are arranged so as to emit light downward to illuminate the bottom of the switching chamber.

In addition, light source substrate 328 is fixed to body 301 by screwing cover 329 to body 301, but the fixing method is not limited thereto. For example, cover 329 may be fitted to body 301.

Cover 329 has a convex shape protruding from ceiling 306a of switching chamber 306.

Cover 329 covering light source substrate 328 is fixed to ceiling 306a of switching chamber 306 with gasket 330 interposed therebetween, so that the gap between cover 329 and ceiling 306a is substantially sealed. In addition, nails are provided around cover 329 to improve the sealing between the cover and the ceiling. In this embodiment, in order to improve the sealing therebetween, an outer nail 331a is provided in the outer circumference of cover 329, and an inner nail 331b is provided in the inner circumference of cover 329. The nails provided at both sides of gasket 330 can further improve the sealing between the cover and the ceiling.

A material forming cover 329 is not particularly limited, but cover 329 is preferably formed of a resin material having high durability for, for example, ultraviolet rays. In addition, a process of forming fine irregularities on the surface to allow the surface to have a polarizing function may be performed on the cover, which makes it possible to improve the diffusibility of light.

Next, the operation and effect of the refrigerator having the above-mentioned structure will be described below.

Only when the user operates operating panel 314 to set the internal temperature of switching chamber 306 to a chilled temperature range or a partial freezing temperature range, ultraviolet LED 326 and blue LED 327 are turned on. However, when the user operates operating panel 314 to set the internal temperature of switching chamber 306 to a freezing temperature range, ultraviolet LED 326 is turned off.

In general, when the ultraviolet rays are emitted to food for a long time, lipid oxidation of food is accelerated, and thus the quality of food deteriorates. However, when the ultraviolet rays are emitted to meat or fishes for a short time, such as one or two weeks, in the chilled or partial freezing temperature range, the radiation of the ultraviolet rays does not cause the deterioration of food, and can obtain an antimicrobial effect.

Since food stored in the chilled or partial freezing temperature range of -3 to 0°C is in a non-frozen state or a partially frozen state, the activity of enzyme protein or genes in the cells is lowered. Therefore, it is possible to delay the proliferation of microorganisms, but it is difficult to completely prevent the proliferation of microorganisms.

Therefore, the ultraviolet ray and the blue light emitted from the LEDs make it possible to inactivate genes of microorganisms, such as mold or bacteria, that are deposited on the surface of food or the inner surface of switching chamber 6, and the proliferation of microorganisms. As a result, the shelf life of food can be improved.

Since the chilled or partial freezing temperature range is generally used to preserve animal food that is easy to decay, such as meat or fishes, the method of improving the antimicrobial effect in these temperature ranges is effective in the actual use of the refrigerator.

When the internal temperature of switching chamber 306 is set to a freezing temperature range, free water contained in the cells of stored food is frozen, and the enzyme protein or genes of food stored in the switching chamber is inactivated. Therefore, the decay of food is prevented. In addition, the proliferation of bacteria or microorganisms deposited on the food is inactivated. Therefore, even when light source 313 is not turned on, there is no fear that microorganisms will be proliferated. For this reason, when the freezing temperature range is set, light source 313 is turned off.

Further, when light is not emitted from the light source in the freezing temperature range, it is possible to reduce energy consumption due to the emission of light. In addition, it is possible to prevent an increase in the internal temperature of the switching chamber due to the emission of light, and thus further improve the shelf life in the freezing temperature range.

As described above, only when a temperature range for preserving food for a short time is set, ultraviolet rays are emitted from the light source, which makes it possible to prevent the lipid oxidation of food due to the emission of light and improve the antimicrobial effect.

The proliferation of microorganisms increases exponentially in a temperature range of 0°C or more. When ultraviolet LED 326 and blue LED 327 are turned on, the internal temperature of the switching chamber is increased by about 4 to 5°C due to heat generated from the LEDs. In this case, the proliferation of microorganisms is accelerated due to the increase in temperature, and the antimicrobial effect by the ultraviolet rays may not be obtained. For this reason, light source 313 is not turned on in the temperature range of 0°C or more. However, when ultraviolet LED 326 and blue LED 327 can emit a sufficient amount of light to obtain the antimicrobial effect capable of preventing the proliferation of microorganisms due to the increase in temperature, light source 313 may be turned on in the temperature range of 0°C or more.

When light source 313 is used in the temperature range of 0°C or more in which the proliferation of microorganisms is accelerated, it is important to set the intensity of light emitted ultraviolet LED 326 at a safety level that is capable of improving the antimicrobial effect and is harmless to the human body.

Since light emitted from blue LED 327 can prevent the proliferation of mold, ultraviolet LED 326 and blue LED 327 need to be turned on at the same time in order to improve the antimicrobial effect.

In this embodiment, in order to improve the antimicrobial performance of switching chamber 306, light source 313 is provided at a substantially central portion of ceiling 306a of switching chamber 306 such that light is emitted downward. Therefore, light emitted from the light source reaches every corner of the bottom of switching chamber 306.

In the embodiment, in order to emit light to every corner of switching chamber 306, fine irregularities are formed on the surface of a portion of cover 329 to allow a portion of cover 329 to have a polarizing function, which makes it possible to improve the diffusibility of light. Alternatively, after performing a process of the cover to have the polarizing performance or providing a polarizing plate on the cover, a transparent portion may be provided in cover 329 to diffuse light to every corner.

Furthermore, cover 329 has a convex shape protruding from ceiling 306a of switching chamber 306, and the convex portion includes a flat portion and an inclined portion. According to this structure, light is emitted to a portion immediately below the lamp through the flat portion, and light is emitted in the horizontal direction of the lamp through the inclined portion.

In order to diffuse light emitted from the lamp in the horizontal direction, the flat portion may be formed such that a part of the flat portion has a polarizing function, and the inclined portion may be formed of a transparent cover having high transmittance. In this case, it is possible to improve the effect of diffusing light in the horizontal direction.

Further, an LED having high directivity may be used to improve the effect of diffusing light in the horizontal direction, without emitting light downward.

Among processes for imparting a polarizing function, the process of forming fine irregularities on the surface of the cover makes parts inside the cover, such as the control substrate and wiring lines formed on the control substrate, invisible to the user, as compared to transparent cover 329. Therefore, the diffusibility of light is improved due to the polarizing effect, and the user is difficult to see the inside of cover 329 since fine irregularities are formed on the surface of a portion of the cover 329 that is generally seen by the user. As a result, it is possible to improve the quality of light source 313.

Cover 329 covering light source substrate 328 is fixed to ceiling 306a of switching chamber 306 with gasket 330 interposed therebetween, so that the gap between the cover and the ceiling is substantially sealed. The cover can prevent a foul odor from the control substrate or the LED from being leaking to the outside. The control substrate or the LED provided inside cover 329 is preferably formed of a material that does not emit an offensive odor to the switching chamber even when the sealing of cover 329 deteriorates, not phenol that emits a four odor.

Next, the antimicrobial effect of animal food when switching chamber 306 according to this embodiment is used will be described below.

Fig. 5 is a graph illustrating the relationship between the amount of light emitted from the ultraviolet LED and the number of microorganisms when a salmon is stored in the switching chamber for one week. Fig. 5 shows the relationship between the number of microorganisms and the amount of light emitted from ultraviolet LED 326 when a salmon, which is a sample selected from food that is generally stored in switching chamber 306 whose internal temperature is set to -1°C, is stored in switching chamber 306 for one week while irradiating the salmon with light emitted from ultraviolet LED 326. In the graph shown in Fig. 5, the horizontal axis indicates the amount of light emitted from ultraviolet LED 326, and the vertical axis indicates the number of microorganisms when the salmon is stored in the switching chamber for one week. As can be seen from Fig. 5, there is significant correlationship between the amount of light emitted from ultraviolet LED 326 and the antimicrobial activity. That is, the higher the intensity of light emitted from ultraviolet LED 326 becomes, the smaller the number of microorganisms becomes.

Further, in the household refrigerator, the intensity of light emitted from ultraviolet LED 326 is preferably set to a safety level that is harmless to the human body, in addition to improving the antimicrobial performance of the refrigerator.

Meanwhile , since food stored in the chilled or partial freezing temperature range is in a non-frozen state or a partially frozen state, the enzyme activity of protein in the cells is maintained. For example, when a fish or meat is stored in the switching chamber, the activity of aminopeptidase contributing to generating a free amino acid is sufficiently high with the decomposition of protein in the cells thereof (not shown). This means that an amino acid, which is a taste component, increases during the preservation period. Therefore, in this embodiment, food is stored in a chilled temperature range or a partial freezing temperature range in which a freezing rate is 70% or less and the growth of the crystal of ice stops at a predetermined point, so that cells are not damaged and the quality of food little deteriorates, and ultraviolet LED 326 emits ultraviolet rays to the food. Therefore, it is possible to prevent the decay of food due to microorganisms and thus improve the shelf life of food. When food is preserved in these temperature ranges, drip can be prevented during thawing, which makes it improve the taste of the food. In addition, the chilled or partial freezing temperature range can accelerate the generation of anthocyanin, which is a red plant pigment that is generally contained in, for example, apples, blueberries, strawberries, green beefsteak plants, broccoli, eggplants, and purple sweet potatoes. In addition, the chilled or partial freezing temperature range can increase the amount of vitamin D in food containing a vitamin D precursor, such as a dried anchovy. As a result, the storage performance of switching chamber 306 is improved.

### (Third example not forming part of the invention)

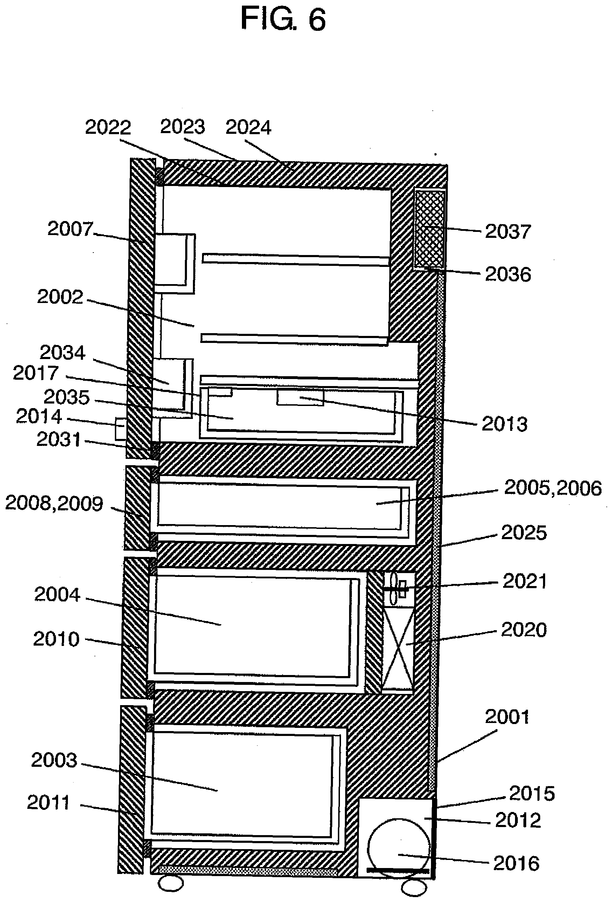

Fig. 6 is a side cross-sectional view illustrating a refrigerator according to a third example not forming part of the invention.

In Fig. 6, body 2001 includes inner case 2022 that is formed of resin, such as ABS, by vacuum forming, outer case 2023 that is made of a metallic material, such as a pre-coat steel sheet, and an insulating wall that is formed by injecting foam insulation 2024 into a space between outer case 2023 and inner case 2022 . For example, rigid urethane foam, phenol foam, or styrene foam may be used as foam insulation 2024. A hydrocarbon-based cyclopentane may be used as a foam material to prevent global warming.

Vacuum insulating material 2025 is closely adhered to the outer case by an adhesive (not shown) in a space between inner case 2022 and outer case 2023 before foam insulation 2024 is foamed. Vacuum insulating material 2025 is formed of a thin plane since it is provided inside the wall of body 2001. In addition, an adhesive, such as a hot-melt adhesive, is coated on the entire surface of vacuum insulating material 2025 to prevent the air from being infiltrated into the adhered portion. Vacuum insulating material 2025 is integrally foamed with foam insulation 2024 to form body 2001. Therefore, vacuum insulating material 2025 has an insulating performance that is 5 to 20 times higher than that of foam insulation 2024 to improve the insulating performance of the insulating wall.

Body 2001 is divided into a plurality of insulating sections, and storage compartments are provided in the divided sections. A hinged door is provided in an upper compartment, and a drawer-type door is provided in a lower compartment. Body 2001 includes refrigerating compartment 2002, drawer-type switching chamber 2006 and drawer-type ice making compartment 2005 that are provided in parallel to each other, drawer-type vegetable compartment 2004, and drawer-type freezing compartment 2003 in this order from the upper side. An insulating door is provided in each of the divided insulating sections with gasket 2031 interposed therebetween. In addition, body 2001 includes hinged door 2007 for the refrigerating compartment, drawer-type door 2008 for the switching chamber, drawer-type door 2009 for the ice making compartment, drawer-type door 2010 for the vegetable compartment, and drawer-type door 2011 for the freezing compartment from the upper side.

Hinged door 2007 is provided with door pocket 2034, which serves as a storage space, and a plurality of storage shelves are provided in refrigerating compartment 2002. Further, storage case 2035 that is comparted by a resin cover is provided at the lowermost part of refrigerating compartment 2002, and a gap between the storage case 2035 and the resin cover is so small that the user cannot put a solid material, such as food, into the case without pulling out the case. Light source 2013 is provided in storage case 2035. The number of light sources 2013 is not limited to one, but a plurality of light sources may be provided. The intensity of light emitted from the light source is not particularly limited. The intensity of light is optimally adjusted by control substrate 2037, and control substrate 2037 may turn on or off the light source, if necessary. At least one light source 2013 is an LED that emits an ultraviolet ray having a wavelength of 280 nm to 400 nm, and light emitted from light source can prevent the proliferation of microorganisms deposited on the surface of food or the inner wall surface of storage case 2035, which makes it possible to improve the shelf life of food. The number of LEDs that emits ultraviolet rays having a wavelength of 280 nm to 400 nm may increase in order to prevent the proliferation of microorganisms deposited on the inner wall of the storage case and thus improve the shelf life. Alternatively, an LED that emits visible light having a wavelength of 400 nm to 800 nm may be provided in addition to the LED that emits ultraviolet rays having a wavelength of 280 nm to 400 nm. In this case, when hinged door 2007 for the refrigerating compartment is opened, the user can visibly recognize the color of visible light emitted from light source 2013 to storage case 2035. The position of light source 2013 in storage case 2035 is not particularly limited. It is preferable that light source 2013 be disposed at a position where light from light source 2013 is not directly emitted to the eyes of the user when the user opens hinged door 2007 for the refrigerating compartment. Alternatively, a cover is provided in light source 2013 to prevent light from being directly emitted to the eyes of the user. A material forming light source 2013 is not particularly limited. For example, a material capable of changing a wavelength may be used to change the color of light emitted only one light source.

A plurality of light sources may be provided in one place in the same compartment, or they may be provided in different places in the same compartment.

The internal temperature of refrigerating compartment 2002 is generally set in the range of 1 to 5°C, which is a lower limit that can store food or other substances without freezing them. However, a user can arbitrarily set the internal temperature of refrigerating compartment 2002 according to the kind of substances. In addition, the internal temperature of refrigerating compartment 2002 may be set to a relatively high temperature of about 10°C in order to preserve wine or vegetables with roots.

In this example, the temperature of storage case 2035 in refrigerating compartment 2002 can be changed to a relatively low temperature, for example, in a temperature range of -3 to 5°C, in order to improve the degree of freshness of fishes, fish-processed goods, and milk products. The internal temperature of vegetable compartment 2004 is generally set to a temperature that is equal to or slightly higher than that of refrigerating compartment 2002, that is, in a temperature range of 2 to 7°C. As the internal temperature of vegetable compartment 504 becomes lower, it possible to maintain the degree of freshness of vegetables for a longer time.

The user can change the internal temperature of switching chamber 2006, which is a temperature changing compartment having a function of changing the internal temperature to a plurality of temperature ranges. The internal temperature of switching chamber 2006 can be changed in a predetermined temperature range from the temperature range of the freezing compartment to a partial freezing temperature and a refrigerating temperature range. The user can operate switch 2014 provided on hinged door 2007 for the refrigerating compartment to adjust the internal temperature of switching chamber 2006, and detector 2017 detects the internal temperature of switching chamber 2006.

Ice making compartment 2005 is an independent ice storage box, and includes an automatic ice making device (not shown) to automatically make ice and stores the ice. The internal temperature of ice making compartment 2005 is set to a freezing temperature range to store ice. However, the internal temperature of ice making compartment 2005 can be set in a temperature range that is slightly higher than the freezing temperature range since its main object is to store ice.

The temperature of freezing compartment 2003 is generally set in the range of -22 to -18°C in order to freeze food. However, the temperature of freezing compartment 2003 can be set in the range of -30 to -25°C in order to keep refrigerated food in a better state.

Body 2001 has machine room 2012 in a recessed portion that is formed at a rear lower side thereof. In addition, body 2001 has second machine room 2036 at the upper side of machine room 2012.

A refrigeration cycle is composed of compressor 2016 provided in machine room 2012, a condenser (not shown), a capillary, serving as a decompression device, and evaporator 2020 that are connected to each other in a ring shape. The forced convection thermal exchange of evaporator 2020 is performed by cooling fan 2021. The condenser (not shown) may be cooled down by a forced air cooling method using a fan, or it may be cooled down by a natural air cooling method by providing the condenser inside outer case 2023 such that heat is transmitted. Alternatively, the condenser may be provided in a partition between insulating doors and connected to a pipe for preventing water droplets.

As described above, compressor 2016 is provided at the rear side of freezing compartment 2003, which is provided at the lowermost part of the body.

In addition, a water-course controller, such as a motor-driven three-way valve, is used to perform the switching of a plurality of evaporators or a plurality of capillaries according to a partitioned structure or a set temperature, and the circulation of cooling gas stops when compressor 2016 is in an off state.

Control substrate 2037 for operating the refrigeration cycle is provided in second machine room 2036 so as to be sealed by a detachable cover. Machine room 2012 is substantially sealed by detachable rear cover 2015.

Evaporator 2020, which is a component of the refrigeration cycle, and cooling fan 2021 are provided at the rear side of vegetable compartment 2004 in the meddle stage.

The operation and effect of the refrigerator having the above-mentioned structure will be described below.

First, the operation of the refrigeration cycle will be described. The refrigeration cycle is operated in response to signals output from control substrate 2037 according to a temperature set in the refrigerator to perform a cooling operation. A high-temperature and high-pressure cooling medium discharged by compressor 2016 is condensed into liquid by the condenser, and the pressure of the condensed liquid is reduced by the capillary, and the low-pressure and low-temperature liquid cooling medium is supplied to evaporator 2020.

Thermal exchange occurs between the cooling medium in evaporator 2020 and the air in the refrigerator by the operation of cooling fan 2021, and the cooling medium is evaporated. A damper (not shown) distributes a low-temperature cooling air to the storage compartments to reduce the internal temperatures thereof. When a plurality of evaporators or decompression devices are used, the water-course controller supplies the cooling medium to necessary evaporators 2020. The cooling medium discharged from evaporator 2020 is supplied to compressor 2016. The refrigeration cycle is repeatedly performed to reduce the internal temperature of the refrigerator.

The temperature of storage case 2035 of refrigerating compartment 2002 can be changed in several stages according to the user' s purpose or favorite. In the third example, the temperature of storage case 2035 can be changed to a partial freezing temperature of about -3°C or a chilled temperature of about 0°C.

The light source used in storage case 2035 having a relatively small storage volume may cause an increase in the temperature of a storage space due to heat generated from the lamp, the acceleration of the deterioration of material forming the storage space, such as resin, due to light emitted from the lamp, or the acceleration of the oxidation of food due to light emitted from the lamp. Therefore, at least one of light sources 2013 is composed of an LED that emits ultraviolet rays having a wavelength of 280 nm to 400 nm, which belongs to the wavelength range of ultraviolet rays A and B, is harmless to the human body, and little accelerates the deterioration of a material or food, and generates little heat, a low running cost, and high durability. Control substrate 2037 adjusts the optimal wavelength and intensity of light emitted from light source 2013, and may control the light source to be turned on at all times. In particular, when storage case 2035 is set to a partial freezing temperature, that is, when food does not need to be completely frozen, it is possible to obtain the antimicrobial effect in a wavelength range of 280 nm to 400 nm, which is the wavelength range of ultraviolet rays A and B, and thus improve the shelf life.

In this way, it is possible to maintain the hygienic conditions inside storage case 2035, and thus delay the discoloration, foul odor, and slime on the surface of food caused by microorganisms. Therefore, the refrigerator can improve the shelf life of food by providing light source 2013 emitting ultraviolet rays, and thus sanitarily store food.

Further, some kinds of mushrooms and fishes contain a lot of precursors of vitamin D. When an ultraviolet ray is emitted to the precursors, molecules of the precursors are excited, and are then transformed into vitamin D. Therefore, the refrigerator can store food while increasing the content of vitamin D in a specific food in storage case 2035 by providing light source 2013 in storage case 2035.

Light source 2013 includes an LED emitting light having a wavelength range of 280 nm to 400 nm, which is the range of relatively safe ultraviolet rays A and B, and an LED that emits visible light having a wavelength of 400 nm or more, and these wavelengths are controlled by the same substrate. It is possible to emit light that is similar to sunlight by controlling the wavelengths of light emitted from the LEDs on the same substrate. The wavelength of light is not limited thereto. For example, when an LED emitting blue light having a wavelength of about 550 nm is used, it is possible to prevent the proliferation of mold. When the LED is used together with an LED emitting ultraviolet rays A and B, it is possible to further improve the shelf life.

Light source 2013 includes an LED emitting having a wavelength range of 280 nm to 400 nm, which is the range of relatively safe ultraviolet rays A and B, and an LED that emits red light having a wavelength of about 650 nm. Therefore, light emitted from light source 2013 can accelerate the generation of anthocyanin, which is a red plant pigment that is generally contained in, for example, apples, blueberries, strawberries, green beefsteak plants, broccoli, eggplants, and purple sweet potatoes. Since anthocyanin is generally contained in vegetables or fruits, the set temperature of storage case 2035 is preferably set to -2°C, which is the freezing point of vegetables or fruits, or more in order to prevent the damage of the cells thereof and preserve vegetables or fruits in a good state.

In addition, it is possible to control the LED to emit visible light, such as red, green, and blue, according to a temperature range set in storage case 2035. A change in the color of light according to the set temperature enables the user to visibly recognize the set temperature of storage case 2035 or a variation in the temperature of storage case 2035. In addition, the user can easily determine what kind of articles is stored in storage case 2035 on the basis of the color of light. Therefore, it is possible to prevent the confusion of the user when the user stores food in storage case 2035, and the user can store food suitable for a set temperature in the storage case.

Light source 2013 is controlled on the basis of the result detected by detector 2017. A temperature sensor is used as detector 2017, and when detector 2017 detects a predetermined temperature, a signal corresponding to the detected result is input to control the light source. For example, when a door switch is used as detector 2017, a control signal may be input when a predetermined time has elapsed after the opening of the door is detected, but the invention is not limited thereto.

In the refrigerator according to this example, switching chamber 2006 is provided below refrigerating compartment 2002 and above vegetable compartment 2004 and freezing compartment 2003. This layout enables a woman having an average height to open the door of switching chamber 2006 and to take out food from switching chamber 2006, without bending her back, which makes it possible for the user to conveniently use the refrigerator. Therefore, the usability of the refrigerator is improved.

According to this example, switching chamber 2006, as is a temperature switching chamber having a function of changing the internal temperature thereof to a plurality of different temperature ranges, is provided in at least one of a plurality of storage compartments provided in body 2001. Refrigerating compartment 2002 other than temperature switching chamber 2006 is provided with a partitioned storage region having a function of changing the temperature thereof in several stages according to the user's purpose or favorite, in addition to a storage region whose temperature is set to a refrigerating temperature range and which includes a plurality of shelves. Storage case 2035 is formed in the partitioned storage region, with an upper side thereof covered with a partition wall made of resin.

The user can arbitrarily set the temperature range of storage case 2035 a general refrigerating temperature range, a partial freezing temperature of about -3°C, or a chilled temperature of about 0°C. An LED, which serves as the light source having the wavelength of the ultraviolet region, is provided in the partitioned storage region having a function of changing the temperature thereof.

According to this structure, switching chamber 2006 can be set to a temperature range that is frequently used. The partitioned storage region having an LED emitting ultraviolet rays as the light source provided to refrigerating compartment 2002 can be used to preserve food for a long time. Since the user can arbitrarily change the temperature of the partitioned storage region having the LED provided therein, the user can use the storage compartments and the storage regions according to purposes. Therefore, the usability of the refrigerator can be improved.

A drawer-type storage case is provided in the partitioned storage region having the LED, which serves as the light source having the wavelength of the ultraviolet regions in refrigerating compartment 2002. The LED, serving as light source 2013, is provided on the outer surface of the storage case facing the body, and at least portion of storage case 2035 facing the LED is formed of a light transmissive resin.

When storage case 2035 has a relatively small storage volume, the internal temperature of the storage case may increase due to heat generated from light source 2013. However, in this example, since the LED, serving as light source 2013, is provided outside storage case 2035, it is possible to prevent an increase in the internal temperature of storage case 2035, and prevent the LED, serving as light source 2013, from being wetted by water droplets due to the increase in the internal temperature.

In this way, it is possible to effectively illuminate the storage case with ultraviolet rays emitted from a light source through a light transmissive case, while maintaining the inside of the storage case at a low temperature. Therefore, it is possible to further improve the storage performance of the storage case, and obtain a sufficient antimicrobial effect using ultraviolet rays.

In this example, the partitioned storage region having the LED, serving as light source 2013, provided therein has a function of changing the temperature thereof. However, according to the type of products, for example, the internal temperature of storage case 2035 provided in the partitioned storage region may not be changed, but fixed to a predetermined value. In this case, for example, food having a short preservation period and requiring an antimicrobial effect using ultraviolet rays and an increase in amino acid is stored in storage case 2035, and general food, such as seasoning, that has a long preservation period and is likely to deteriorate due to ultraviolet rays is stored in storage regions other than storage case 2035 in refrigerating compartment 2002.

As described above, when the internal temperature of storage case 2035 is set to a predetermined value, that is, a temperature range that is slightly lower than the refrigerating temperature of storage regions outside storage case 2035 in refrigerating compartment 2002, such as a partial freezing temperature of about -3°C or a chilled temperature range of about 0°C, the antimicrobial effect by ultraviolet rays and an increase in amino acid are further improved, as compared to the refrigerating temperature range. When the partitioned storage region having the light source provided therein does not have a temperature changing function, it is preferable that the partitioned storage region be set to a temperature range slightly lower than the refrigerating temperature range in order to further improve the antimicrobial effect by the ultraviolet rays.

In the refrigerator according to this example, considering the usability of the refrigerator, a hinged door is provided in refrigerating compartment 2002, and drawer-type doors are provided in the other storage compartments. However, the invention is not limited thereto.

As described above, in refrigerating compartment 2002 according to this example, light source 2013 including an LED emitting light having a wavelength range of 280 nm to 400 nm, which is the range of relatively safe ultraviolet rays A and B, and an LED, which serves as the visible light source having a wavelength of about 400 nm or more is provided in storage case 2035. Therefore, storage case 2035 can prevent an increase in temperature when food is put into or taken out from the storage case, and prevent the proliferation of various bacteria. As a result, it is possible to improve the antimicrobial effect even when light source 2013 emits light having a long wavelength.

### (Fourth example not forming part of the invention )

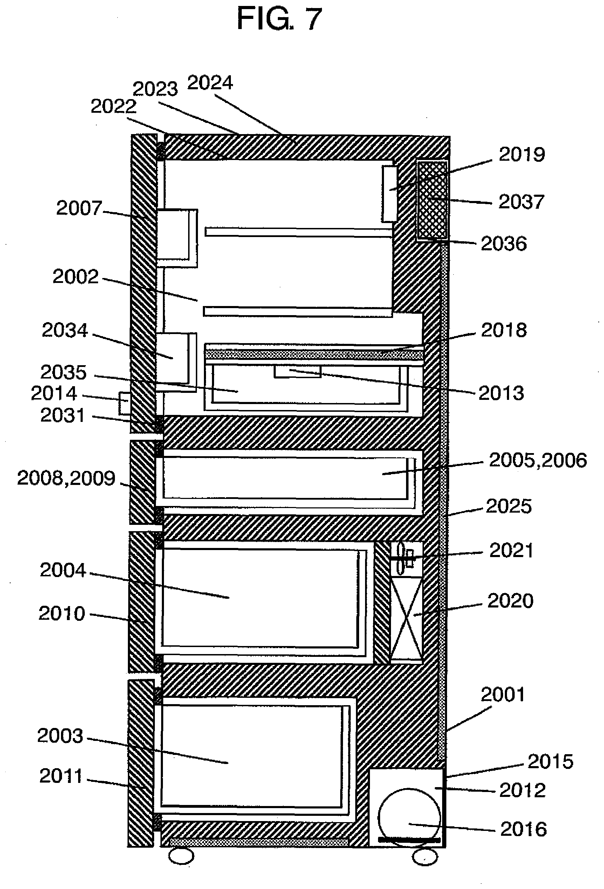

Fig. 7 is a side cross-sectional view illustrating a refrigerator according to a fourth example not forming part of the invention.

In Fig. 7, insulating material 2018 is provided at the boundary between storage case 2035 and refrigerating compartment 2002. Since insulating material 2018 improves the thermal insulating property of storage case 2035, it is possible to prevent an increase in the internal temperature of storage case 2035 even when hinged door 2007 of the refrigerating compartment is opened, and thus maintain the quality of food. In addition, insulating material 2018 can reduce the transmission of heat between the partitioned regions. As a result, it is possible to prevent unnecessary energy consumption and an increase in the amount of energy consumed by body 2001.

Since the storage case is partitioned by insulating material 2018, it is possible to prevent ultraviolet rays from being incident on other regions, and thus discriminatively store food in a region to which the ultraviolet rays are emitted or a region to which no ultraviolet rays are emitted.

When insulating material 2018 is coated with a light shielding material, a light shielding member can prevent the scattering of light to the outside. As a result, the amount of light emitted to storage case 2035 increases, and the shelf life is improved.

### (Fifth example not forming part of the invention)

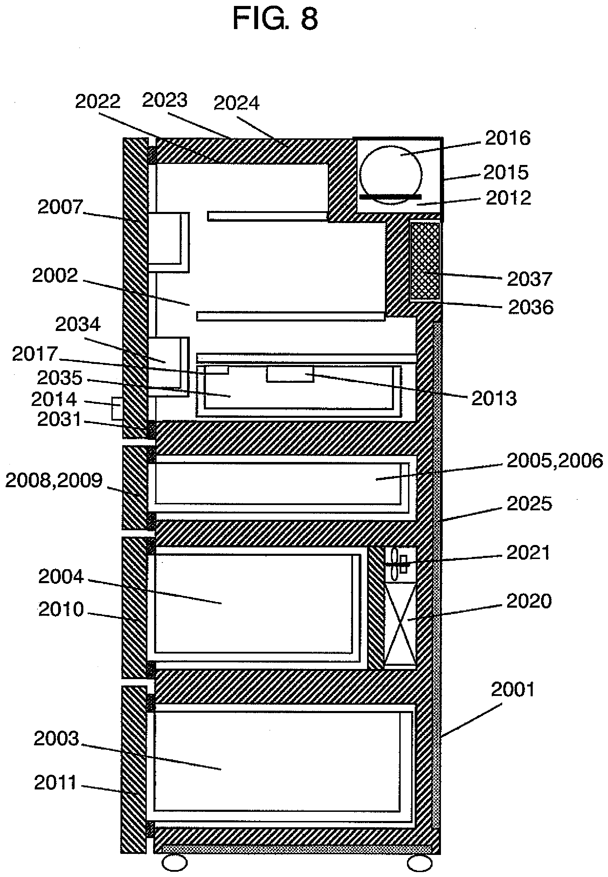

Fig. 8 is a side cross-sectional view illustrating a refrigerator according to a fifth example not forming part of the invention.

In this example, the same components as those in the refrigerator according to the third and fourth examples are denoted by the same reference numerals, and a detailed description thereof will be omitted.

In Fig. 8, light source 2013 is provided in storage case 2035. Machine room 2012 housing the main components of a refrigeration cycle, such as compressor 2016 and a condenser (not shown), is provided at a rear upper side of refrigerating compartment 2002.

In the related art, since machine room 2012 is arranged on the rear side of freezing compartment 2003, which is the lowest part of body 2001, machine room 2012 reduces the volume of freezing compartment 2003. However, in this example, machine room 2012 is provided at the rear upper side of refrigerating compartment 2002, which is a place that has not been used in the refrigerator according to the related art since it is too high to touch. Therefore, a space corresponding to machine room 2012 according to the related art can be used as a storage space, which makes it possible to increase the effective volume of freezing compartment 2003 without increasing the size of a refrigerator.

Further, in the related art, it is difficult to take out articles stored at rear corners of the uppermost shelf of refrigerating compartment 2002. However, since machine room 2012 causes the depth of the shelf to be reduced, the user can easily view the articles and take out them. That is, the machine room makes it possible for the user to easily take out articles put on the shelf in refrigerating compartment 2002, and thus the time required to open or close hinged door 2007 of refrigerating compartment 2002 can be shortened. As a result, it is possible to prevent an increase in the internal temperature of refrigerating compartment 2002, and improve the shelf life of food stored in refrigerating compartment 2002. Therefore, the storage performance of storage case 2035 is improved by only providing light source 2013 including an LED emitting light having a wavelength range of 280 nm to 400 nm, which is the range of relatively safe ultraviolet rays A and B, and an LED that emits visible light having a wavelength of 400 nm or more in storage case 2035.

In this example, body 2001 has machine room 2012 that is a concave portion provided at a rear upper side of refrigerating compartment 2002, which is an uppermost storage compartment, and a compressor is provided in machine room 2012. Since the compressor is provided at a rear side of the ceiling of the uppermost storage compartment, which is a space that is beyond user's reach and is difficult to use, it is possible to expand a storage space that the user can conveniently use, and thus considerably improve the usability of a refrigerator.

### (Sixth example not forming part of the invention )

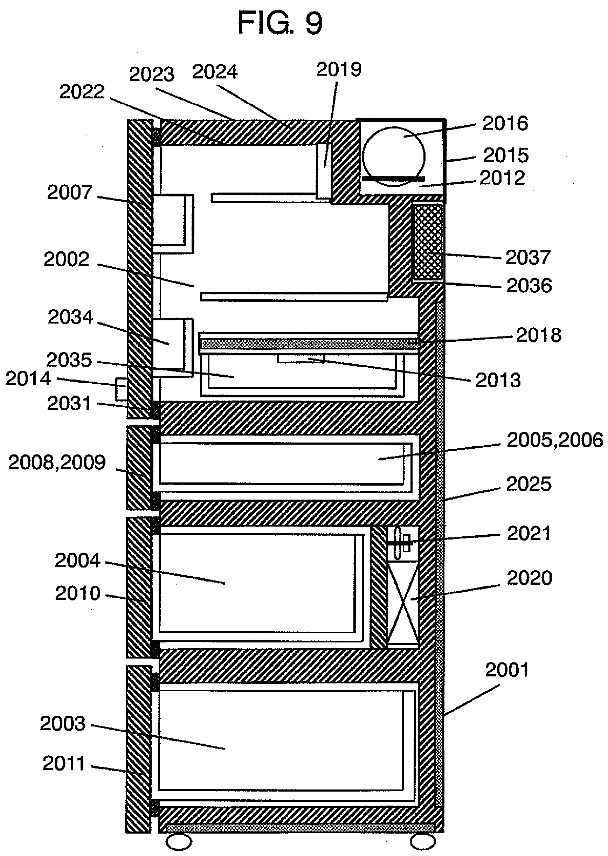

Fig. 9 is a side cross-sectional view illustrating a refrigerator according to a sixth example not forming part of the invention.

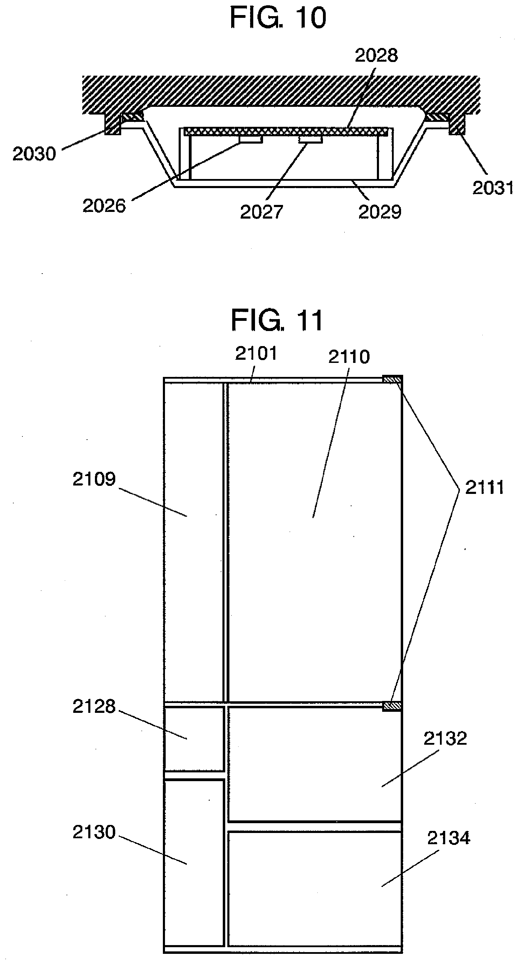

Fig. 10 is a cross-sectional view illustrating a light source according to the sixth example of the invention.

In this example, the same components as those in the third to fifth examples are denoted by the same reference numerals, and a detailed description thereof will be omitted.

In Figs. 9 and 10, insulating material 2018, which is a light-shielding material, is provided at the boundary between storage case 2035 and refrigerating compartment 2002. Machine room 2012 having the main components of a refrigeration cycle, such as compressor 2016 and a condenser (not shown), therein is provided at a rear side of the ceiling of body 2001.

Light source 2013 includes ultraviolet LED 2026 which emits an ultraviolet ray having a peak wavelength of 387 nm, blue LED 2027 which emits a blue light component having a peak wavelength of 468 nm, light source substrate 2028 having LEDs 2026 and 2027 mounted thereon, and cover 2029 which covers the light source substrate. Therefore, the user cannot directly view light source 2013. In addition, light source 2013 is fixed to the ceiling of storage case 2035 by, for example, screws (not shown) so as to face downward, so that LEDs 2026 and 2027 emit light to the bottom of the storage case. For example, cushion 2030 provided between cover 2029 and storage case 2035, and nails 2031 provided around cover 2029 prevent water droplets from being infiltrated into light source 2013.

Insulating material 2018 is provided at the boundary between storage case 2035 and refrigerating compartment 2002, and improves the thermal insulating performance of storage case 2035. Therefore, it is possible to prevent an increase in the internal temperature of storage case 2035 even when hinged door 2007 of the refrigerating compartment is opened, and thus maintain the quality of food. In addition, insulating material 2018 can reduce the transmission of heat between the partitioned regions. As a result, it is possible to prevent unnecessary energy consumption and an increase in the amount of energy consumed by body 1.

In this example, insulating material 2018, which is a light shielding material, is provided on the ceiling of the partitioned storage region in which storage case 2035 including light source 2013 is provided. The light shielding material prevents ultraviolet rays emitted from light source 2013 from being scattered to the outside, and the ultraviolet rays are intensively emitted to the inside of storage case 2035. As a result, the ultraviolet rays can further improve the antimicrobial effect inside storage case 2035.

In this example, the insulating material is used as a light shielding material to obtain both the light shielding effect and the thermal insulating effect. However, when significant thermal insulating effects are not needed, for example, an opaque resin, a colored resin, or a metallic material may be used as the light shielding material.

When a metallic material having high reflectance is used, it is possible to further increase the amount of light emitted to storage case 2035, and diffuse light to every corner of storage case 2035.

For example, in this example, one surface of insulating material 2018 facing storage case 2035 may be coated with a reflective material. In this case, it is possible to reflect light to the storage case. Therefore, the amount of light inside storage case 2035 can increase, and light can be emitted to every corner of the storage case. As a result, the antimicrobial effect by ultraviolet rays can be further improved, and the shelf life of food can be improved.

### (Seventh example not forming part of the invention )

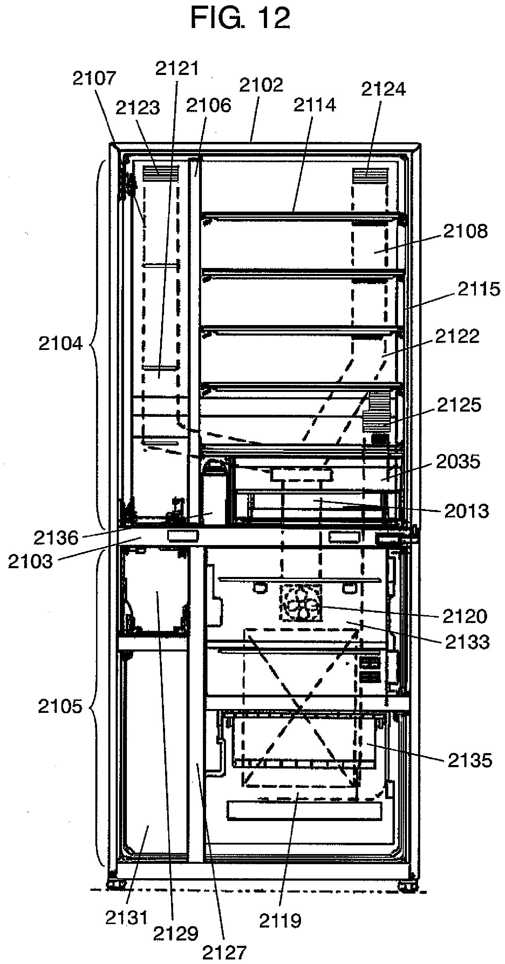

Fig. 11 is a front view illustrating a refrigerator according to an seventh example not forming part of the invention, and Fig. 12 is a front view illustrating the refrigerator according to the seventh example with doors being removed.

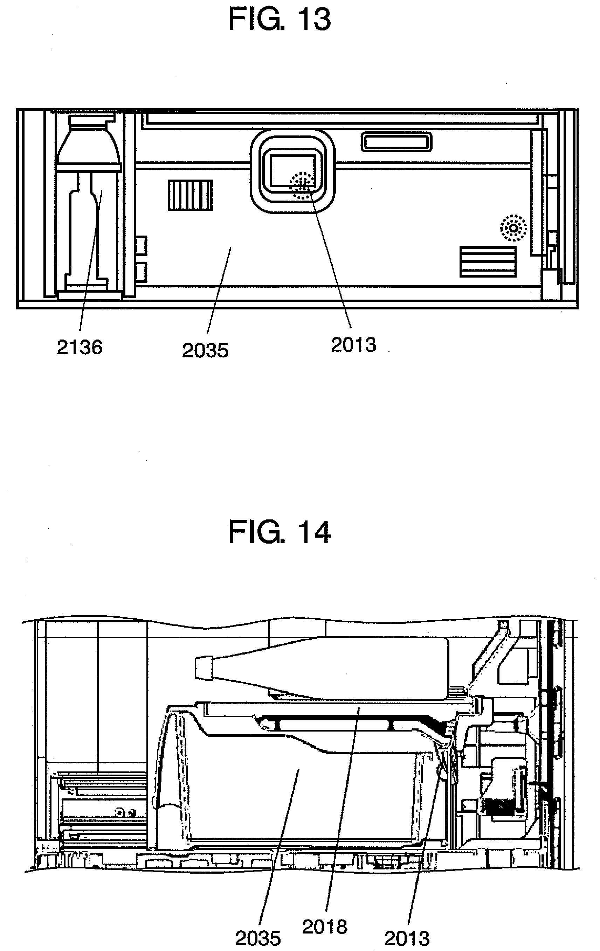

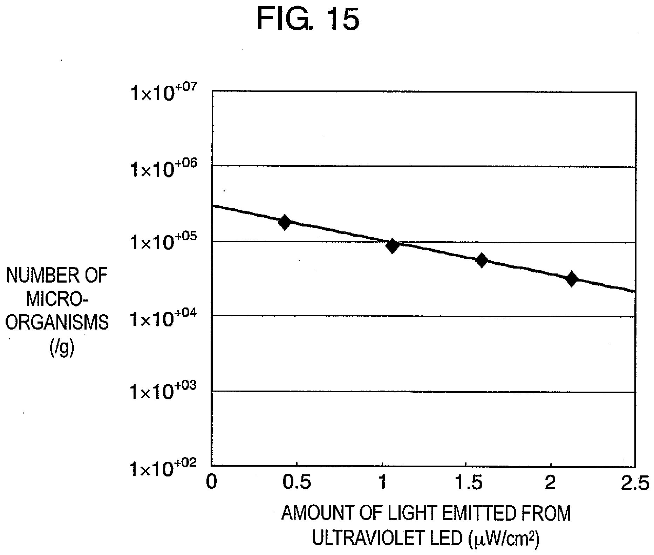

Fig. 13 is a partial enlarged front view illustrating a storage case according to the seventh example , and Fig. 14 is a partial enlarged cross-sectional view illustrating the storage case according to the seventh example. Fig. 15 is a graph illustrating the relationship between the amount of ultraviolet rays emitted from an LED and the number of microorganisms when a salmon is stored for one week in the switching chamber according to the seventh example.

In Figs. 11 to 14, body 2101 includes insulating case 2102 and a plurality of doors, which will be described below, and partition wall 2103 partitions the body into upper compartment 2104 and lower compartment 2105. Upper compartment 2104 is divided into left region 2107 and right region 2108 by first partition wall 2106. A front opening of left region 2107 is closed by drawer-type door 2109, and a front opening of right region 2108 is closed by hinged door 2110. The width of drawer-type door 2109 is smaller than the width of hinged door 2110, and thus the volume of left region 2107 is smaller than the volume of right region 2108. Hinge portion 2111 is provided on one side of hinged door 2110 opposite to drawer-type door 2109. Upper compartment 2104 is a refrigerating compartment in which left region 2107 and right region 2108 are set to a refrigerating temperature. A plurality of trays are provided in left region 2107 that is closed by drawer-type door 2109. When drawer-type door 2109 is opened, the trays are also pulled out.

A plurality of shelves 2114 on which articles are placed are provided in right region 2108 that is closed by hinged door 2110 opposite to drawer-type door 2109. One side edge of each shelf is supported and fixed to a bracket of inner case 2115 of the insulating case. The other side edge of each shelf is supported and fixed to a bracket that is provided on the side surface of first partition wall 2106.

Storage case (partitioned storage region) 2035 is a partitioned storage area that is partitioned by a resin cover and can change the internal temperature thereof to a refrigerating temperature range of 1 to -3°C that is suitable for preserving, for example, meat and fishes, a chilled temperature of about 0°C, and a partial freezing temperature range of about -3°C that is slightly lower than the refrigerating temperature range. Light source 2013 is provided in storage case (partitioned storage region) 2035. Light source 2013 is provided at the uppermost part of the rear surface of storage case 2035. Light source 2013 is provided so as be slightly inclined in the downward direction with respect to the vertical direction of the rear surface. Therefore, light emitted from light source 2013 to food stored in the storage case becomes intensive, and the shelf life of food is improved. Light source 2013 is provided such that light from the light source is not directly emitted to the eyes of the user when hinged door 2110 is opened, considering the safety of the user.

Lower compartment 2105 is divided into a left region and a right region by second partition wall 2127 in the horizontal direction. The left region includes ice making compartment 2129 having drawer-type door 2128, and freezing compartment 2131 having drawer-type door 2130 in this order from the upper side, and the right region includes vegetable compartment 2133 having drawer-type door 2132 and freezing compartment 2135 having drawer-type door 2134.

The operation and effect of the refrigerator having the above-mentioned structure will be described below.

In recent years, with an increase in the size of refrigerators, the number of beverage PET bottles or dressing bottles stored in the refrigerator has increased. In general, these bottles are stored in a storage space provided on the rear surface of a hinged door of the refrigerator. According to the survey, the user frequently opens the refrigerating compartment in order to take out these bottles.

When the number of times the user opens the door to take out these bottles increases, the internal temperature of the refrigerating compartment increases, and particularly, in summer, the quality of food stored in the refrigerating compartment deteriorates due to the increase in the internal temperature. In order solve this problem, in this example, trays are provided in the left region of the refrigerating compartment that is divided by partition wall 2106 such that they are pulled out when the drawer-type door of the left region is opened. According to this structure, it is possible to discriminatively store various types of bottles, such as PET bottles, beverage bottles, and cans, according to their types, and thus to prevent an increase in the temperature of food stored in the storage space provided on the rear surface of hinged door 2110 even when bottles are taken out frequently. As a result, the shelf life of food is improved, and energy consumption is reduced. In addition, in many cases, for example, meat or fishes are stored in storage case (partitioned storage region) 2035 that is provided at the lowermost part of hinged door 2110 so as to be partitioned by a resin cover, and is set to a refrigerating temperature range. In this food, microorganisms are likely to be proliferated due to an increase in temperature caused by the frequent opening of the door. However, according to this example, while preventing an increase in the internal temperature of storage case 2035 occurring when the user frequently opens the door to take out, for example, PET bottles, at least one light source 2013, which is an LED that emits ultraviolet rays having a wavelength of 280 nm to 400 nm is provided, which makes it possible to prevent the proliferation of microorganisms deposited on the surface of food or the inner wall surface of storage case 2035, and thus further improve the shelf life of food. The number of light sources 2013 is not limited to one, but a plurality of light sources 2013 may be provided. That is, it is possible to increase the number of LEDs that emit ultraviolet rays having a wavelength of 280 nm to 400 nm in order to prevent the proliferation of microorganisms deposited on the inner wall surface of storage case 2035 and to improve the shelf life of food. An LED that emits visible light may be provided in addition to the LED that emits ultraviolet rays having a wavelength of 280 nm to 400 nm.

For example, ultraviolet rays and blue light may be emitted at the same time. In this case, it is possible to prevent the proliferation of eumycetes, such as mold, and thus further improve the shelf life of food. The amount of light emitted to the storage case is not limited to a specific value. The optimal intensity of light emitted is adjusted by control substrate (not shown), and the control substrate turns on or off the light source, if necessary.

For example, the light source may be turned on when hinged door 2110 is opened. However, when the drawer-type door is opened, a light source that is provided at a position where light is not emitted to the eyes of the user may be kept in an on state. In particular, light including an ultraviolet range is invisible to the human eye. Therefore, when the door is opened, the LED emitting ultraviolet rays is turned off, and the LED emitting safe light, such as visible light, is kept in an on state. In this way, the user can recognize the color of light having a safe wavelength range, and light emitted from the LED that is turned on when the door is closed can increase the amount of amino acid. As a result, it is possible to improve the quality of the refrigerator.

The position of light source 2013 in storage case 2035 is not particularly limited. It is preferable that light source 2013 be disposed at a position where light from light source 2013 is not directly emitted to the eyes of the user when the user opens hinged door 2110 for the refrigerating compartment. For example, in the case of refrigerators used in Japanese, when a Japanese woman having an average height stands upright and opens hinged door 2110, light source 2013 is preferably arranged at a position where light emitted from the light source is not directly emitted to the eyes of the woman. When refrigerators are used in other foreign countries, it is preferable that the position of the light source be determined according to the average height of women of the country.

Alternatively, light source 2013 may be covered with a cover to prevent light from being directly emitted to the eyes of the user. A material forming the cover of light source 2013 is not particularly limited. A material that is subjected to a process of forming fine irregularities on the surface thereof to have a function of partially polarizing light may be used as the material forming the cover. In this case, it is possible to improve the diffusibility of light. In addition, the positions of a plurality of light sources are not limited to one place in the same compartment, but the light sources may be provided in different places in the same compartment.

From this viewpoint, in this example, an insulating material having a light shielding property is provided at an upper part of the light source, and light source 2013 is provided such that light is not directly emitted to the eyes of the user. In addition, the difference between the internal and external temperatures of storage case 2035 is maintained by the insulating material. In this way, the storage performance of storage case 2035 is improved.

As described above, the light shielding material prevents ultraviolet rays emitted from light source 2013 from being scattered to the outside, and the ultraviolet rays are intensively emitted to the inside of storage case 2035. As a result, the antimicrobial effect by the ultraviolet rays in storage case 2035 can be improved.

Further, in this example, the insulating material is used as a light shielding material to obtain both the light shielding effect and the thermal insulating effect. However, when significant thermal insulating effects are not needed, for example, an opaque resin, a colored resin, or a metallic material may be used as the light shielding material.

When a metallic material having high reflectance is used, it is possible to further increase the amount of light emitted to storage case 2035, and diffuse light to every corner of storage case 2035.

For example, in this example, at least one surface of insulating material 2018 facing storage case 2035 may be coated with a reflective material. In this case, it is possible to reflect light inside storage case 2035. Therefore, the amount of light inside storage case 2035 can increase, and light can be emitted to every corner of the storage case. As a result, the antimicrobial effect by ultraviolet rays can be further improved, and the shelf life of food can be improved.

Specifically, insulating material 2018 is provided at the boundary between storage case 2035 and the refrigerating compartment. Since insulating material 2018 improves the thermal insulating property of storage case 2035, it is possible to prevent an increase in the internal temperature of storage case 2035 even when hinged door 2110 of the refrigerating compartment is opened, and thus maintain the quality of food. In addition, insulating material 2018 can reduce the transmission of heat between the partitioned regions. As a result, it is possible to prevent unnecessary energy consumption and an increase in the amount of energy consumed by the body. Since the storage case is partitioned by insulating material 2018, it is possible to prevent ultraviolet rays from leaking to adjacent partitioned regions, and discriminatively use a region in which the ultraviolet rays are emitted and a region in which no ultraviolet rays are emitted according to the purposes.

Furthermore, at least one surface of insulating material 2018 facing storage case 2035 is coated with a reflective material. Therefore, the insulating material having a light shielding property can prevent light from being scattered to the outside. Since a material having high reflectance is provided at the upper part of storage case 2035, the amount of light emitted to storage case 2035 can increase, and the shelf life of food can be improved.

The insulating material and the light shielding plate provided at the upper part of storage case 2035 can increase the intensity of light emitted to from the light source 2013, and improve the shelf life of food.

In this example, drawer-type storage case 2035 is provided in the partitioned storage region in which an LED emitting the light of the wavelength of the ultraviolet regions is provided as the light source 2013 in right region 2108 of the refrigerating compartment. Light source 2013 is provided on the outer surface of storage case 2035 facing the body, and at least a portion of storage case 2035 opposite to light source 2013 is formed of a light transmissive material.

When storage case 2035 has a relatively small storage volume, the internal temperature of storage case 2035 may increase due to heat generated from the light source. However, in this example, since light source 2013 is provided outside storage case 2035, it is possible to prevent an increase in the internal temperature of storage case 2035, and prevent light source 2013 from being wetted by water droplets due to the increase in the internal temperature. In this way, it is possible to effectively illuminate the storage case with ultraviolet rays emitted from the light source through a transparent case, while maintaining the inside of storage case 2035 at a low temperature. Therefore, it is possible to further improve the storage performance of storage case 2035, and obtain a sufficient antimicrobial effect using ultraviolet rays.

In this example, storage case 2035 is adjacent to ice making water supply tank 2136. There is a fear that various bacteria will be proliferated in ice making water supply tank 2136. When general water, not chlorodized water, is used, the decay of water due to various bacteria becomes remarkable, as compared to tap water. Storage case 2035 and water supply tank 2136 may be formed of a material having high transmittance. In this case, light emitted from light source 2013 can prevent the proliferation of bacteria in water supply tank 2136. That is, it is possible to prevent the proliferation of microorganisms in regions adjacent to storage case 2035 by improving the intensity of light emitted from light source 2013 or the diffusibility of light emitted from light source 2013.

In this example, body 2101 includes a plurality of thermally insulated storage compartments. Among the plurality of storage compartment, upper compartment 2104, which is an uppermost storage compartment, is divided into left region 2107 and right region 2108, and two doors are provided to close up two divided front openings of the refrigerating compartment including left region 2107 and right region 2108. That is, drawer-type door 2109 is provided to close up a front opening of left region 2107, and hinged door 2110 is provided to close up a front opening of right region 2108. Among the two doors, a partitioned storage region having an LED, serving as light source 2013, provided therein is formed in a projection plane in front of hinged door 2110 that closes up the front opening of right region 2108.

In this way, when the user takes out articles from the partitioned storage region having light source 2013 provided therein, the user can open only the door corresponding to the partitioned storage region having light source 2013 provided therein, without opening two doors. Therefore, the user can open the door with one hand, and thus the usability of the refrigerator can be improved.

In this example, body 2101 includes a plurality of thermally insulated storage compartments. Among the plurality of storage compartment, upper compartment 2104, which is an uppermost storage compartment, is divided into left region 2107 and right region 2108, and two doors are provided so as to close up two divided front openings of the refrigerating compartment including left region 2107 and right region 2108. The refrigerating compartment is divided into left region 2107 and right region 2108 by an engaging portion between the two doors, and a plurality of partitioned storage regions are provided in right region 2108, which is one of the two divided regions. Storage case 2035 is provided in one of the plurality of partitioned storage regions in which the LED, serving as light source 2013, is provided.

According to this structure, the uppermost refrigerating compartment having a larger storage volume than the other storage compartments is divided into a left region and a right region, and doors are provided to correspond to the divided regions. Therefore, the user can open only the door of the storage region having an article to be taken out stored therein, with the door of the other storage region being closed. As a result, it is possible to prevent a considerable increase in the internal temperature of the storage region due to the opening of the uppermost storage compartment and the infiltration of bacteria from the outside of the refrigerator, and thus prevent the deterioration of the quality of food stored in the uppermost storage compartment.

In this example, among the two doors that close up two divided front openings of the refrigerating compartment, drawer-type door 2109 having a plurality of trays is provided as a door that has a smaller width in the horizontal direction and closes up the front opening of left region 2107. When drawer-type door 2109 is opened, the plurality of trays are also pulled out.

In the related art, a door having a small width in the horizontal direction makes it difficult for the user to take out stored articles, and actually, it is very difficult for the user to open only the door having small width to take out the stored articles. However, in this example, since drawer-type door 2109 is used as the door having a small width, the user can open only the door having a small width to easily take out the stored articles. In particular, beverage bottles, such as PET bottles that are inconvenient to store, or seasoning cases can be stored in the drawer-type storage compartment, although they are frequently used, and thus the user can easily discriminate the types of bottles. In addition, it is possible to prevent an increase in the internal temperature of the storage compartment closed by the door having a large width. As a result, the usability of the uppermost storage compartment can be improved, and the shelf life of food can be improved.

In addition, the plurality of trays are integrally pulled out with drawer-type door 2109. Therefore, when drawer-type door 2109 is opened, the inside of the storage compartment is viewed from the side of drawer-type door 2109, which makes it possible for the user to conveniently take out stored articles.

In this way, beverage bottles, such as PET bottles that are difficult to store, or seasoning cases are stored in the drawer-type storage compartment, which is left region 2107, although they are frequently used, and thus the number of times hinged door 2110 provided in right region 2108 is opened is considerably reduced. As a result, it is possible to prevent an increase in the temperature of storage case 2035 provided in the partitioned storage region having light source 2013 provided therein, and further improve the shelf life of food using ultraviolet rays emitted from light source 2013.

Next, the antimicrobial effect of animal food when storage case 2035 according to this example is used will be described below. Fig. 15 is a graph illustrating the relationship between the amount of light emitted from an ultraviolet LED and the number of microorganisms when a salmon is stored for one week in the storage case according to the seventh example. Fig. 15 shows the relationship between the number of microorganisms and the amount of light emitted from the ultraviolet LED when a salmon, which is a sample selected from food that is generally stored in storage case 2035 whose internal temperature is set to -1°C, is stored in storage case 2035 for one week while changing irradiation to the salmon with light emitted from light source 2013. In the graph shown in Fig. 15, the horizontal axis indicates the amount of light emitted from the ultraviolet LED, and the vertical axis indicates the number of microorganisms after the salmon is stored in the storage case for one week. As can be seen from Fig. 15, there is significant correlationship between the amount of light emitted from the ultraviolet LED and the antimicrobial activity. That is, the higher the intensity of light emitted from the ultraviolet LED becomes, the smaller the number of microorganisms becomes.

An ultraviolet emission antimicrobial activity is evaluated using colon bacilli (Escherichia coli IFO 3972), with reference to a photocatalytic antimicrobial test III (2003) prescribed by Society of Industrial-Technology for Antimicrobial Articles (SIAA) . According to the result of the evaluation, comparing the light emission conditions of light source 2013 with dark conditions not using light source 2013 (comparative region), the number of microorganisms under the dark condition is 100 times larger than that under the light emission conditions, and an antimicrobial activity value of 2.03 is obtained. The evaluation proves that an antimicrobial effect is obtained (not shown).

Meanwhile, in the household refrigerator, in addition to the antimicrobial performance, it is preferable that the intensity of light emitted from the ultraviolet LED be set to a level that is harmless to the human body and causes no problem in the safety of the user. Since food stored in the chilled or partial freezing temperature range is in a non-frozen state or a partially frozen state, the enzyme activity of protein in the cells is maintained. For example, when a fish or meat is stored in the refrigerator, the activity of aminopeptidase contributing to generating a free amino acid is sufficiently high with the decomposition of protein in the cells thereof (not shown). This means that an amino acid, which is a taste component, increases during the preservation period. In this example, food is stored in a partially frozen state in which a freezing rate is 70% or less, and thus the growth of the crystal of ice stops at a predetermined point. Therefore, food is stored in the partial freezing temperature range or the chilled temperature range in which cells are not damaged and the quality of food little deteriorates, and the ultraviolet LED emits the lights of the wavelength of the ultraviolet regions to the food. Thus, it is possible to prevent the decay of food due to microorganisms and thus improve the shelf life of food. When food is preserved in these temperature ranges, drip can be prevented during thawing, which makes it improve the taste of the food.

In addition, the chilled or partial freezing temperature range can accelerate the generation of anthocyanin, which is a red plant pigment that is generally contained in food other than meat or fishes, such as, apples, blueberries, strawberries, green beefsteak plants, broccoli, eggplants, and purple sweet potatoes. In addition, the chilled or partial freezing temperature range can increase the amount of vitamin D in food containing a vitamin D precursor, such as a dried anchovy. As a result, the storage performance of switching chamber 2006 can be improved.

Further, in this example, the partitioned storage region having the LED, serving as light source 2013, provided therein has a function of changing the temperature thereof. However, according to the type of products, for example, the internal temperature of storage case 2035 provided in the partitioned storage region may not be changed, but fixed to a predetermined value. In this case, for example, food having a short preservation period and requiring an antimicrobial effect by ultraviolet rays and an increase in amino acid is stored in storage case 2035, and general food, such as seasoning, that has a long preservation period and is likely to deteriorate due to ultraviolet rays is stored in storage regions other than storage case 2035 in the refrigerating compartment.

As described above, when the internal temperature of storage case 2035 is set to a predetermined value, that is, a temperature range that is slightly lower than the refrigerating temperature of storage regions outside storage case 2035 in the refrigerating compartment, such as a partial freezing temperature of about -3°C or a chilled temperature range of about 0°C, the antimicrobial effect by ultraviolet rays and an increase in amino acid are further improved, as compared to the refrigerating temperature range. When the partitioned storage region having the light source provided therein does not have a temperature changing function, it is preferable that the partitioned storage region be set to a temperature range slightly lower than the refrigerating temperature range in order to further improve the antimicrobial effect by the ultraviolet rays.

### (Eighth example not forming part of the invention )

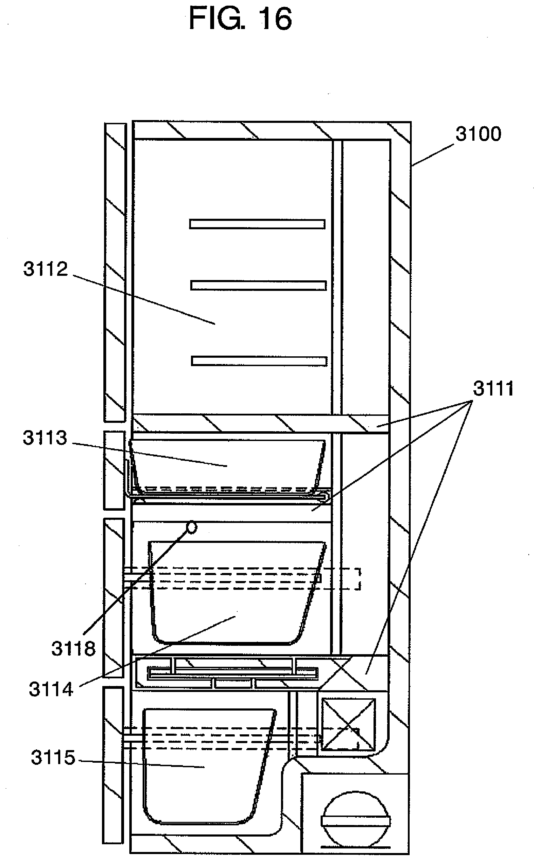

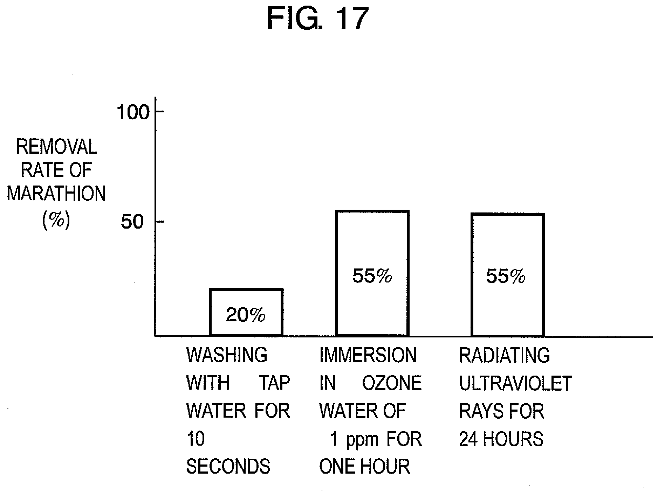

Fig. 16 is a side cross-sectional view illustrating a refrigerator according to an eighth example not forming part of the invention, and Fig. 17 is a diagram illustrating an agricultural chemical removing performance of ultraviolet rays of the refrigerator according to the eighth example not forming part of the invention.

In Fig. 16, refrigerator 3100 is divided into refrigerating compartment 3112, switching chamber 3113, vegetable compartment 3114, and freezing compartment 3115 by partition plates 3111 in this order from the upper side. The inner wall of vegetable compartment 3114 is formed of resin. Ultraviolet lamp 3118 is provided on the ceiling of vegetable compartment 3114. Ultraviolet lamp 3118 provided on the ceiling of vegetable compartment 3114 is an ultraviolet LED that emits ultraviolet rays having a peak wavelength of about 350 nm.

The operation and effect of the refrigerator having the above-mentioned structure will be described below.

An LED emitting light having a wavelength range of 280 nm to 380 nm, which is the range of relatively safe ultraviolet rays A and B, is used as light source 3118. In a general household refrigerator, since an LED emitting ultraviolet rays having a short wavelength is harmful to the human body, it is undesirable to use the LED for the refrigerator. When an LED emitting light having an excessively long wavelength is used, the decomposition capability of agricultural chemicals is lowered. However, light having the above-mentioned wavelength range can be easily absorbed to agricultural chemicals, and thus decompose the agricultural chemicals deposited on vegetables stored in the vegetable compartment, which makes it possible to reduce agricultural chemicals deposited on the stored articles.

The ultraviolet rays having a wavelength range of 280 nm to 380 nm according to this example are included in sunlight, and are harmless to the human body. Therefore, the ultraviolet rays can be continuously emitted to break chemical bonds between agricultural chemical components deposited on the articles stored in the vegetable compartment, and thus decompose and reduce agricultural chemicals while ensuring the safety of the human body. Thus, it is possible to provide the safety of the user and offer vegetables that are harmless to the human body. Since the ultraviolet rays have a wavelength included in sunlight, the ultraviolet rays are harmless to the human body, and can decompose and reduce agricultural chemicals deposited on stored articles, such as vegetables, without deteriorating the quality of vegetables and changing the colors of vegetables.

Further, since the ultraviolet rays have a wavelength range that is harmless to both the human body and stored articles, the ultraviolet rays can be continuously emitted. The longer the radiation time of ultraviolet rays becomes, the larger the agricultural chemicals are decomposed. Therefore, the ultraviolet rays can be emitted for a long time when food is stored in the refrigerator for a long time. In this case, it is possible to decompose a larger amount of agricultural chemicals.

Since an LED is used as ultraviolet lamp 3118, ultraviolet lamp 3118 has a low running cost and high durability in a low temperature environment, such as at a low refrigerating temperature. Therefore, since the ultraviolet lamp can be semi-permanently used, it has high reliability for a long time, high compatibility, and a simple structure. In addition, the ultraviolet lamp can reduce the agricultural chemicals deposited on articles stored in the refrigerator without using a dedicated device.

Since ultraviolet rays can break chemical bonds between agricultural chemicals deposited on the stored articles, they can change, for example, P=O into P-O, and thus can change a hydrophobic double bond to a hydrophilic single bond. In this example, a character 'P' indicates phosphorous, and a character '0' indicates oxygen.

Since the ultraviolet rays cause agricultural chemicals to have a hydrophilic property, the user washes vegetables in water to reduce a larger amount of agricultural chemicals remaining on the vegetables after the radiation of the ultraviolet rays, as compared to the case in which the user washes vegetables to which no ultraviolet rays are emitted in water.

In addition, for example, in some kinds of mushrooms containing a lot of precursors of vitamin D, when the ultraviolet ray is emitted to the precursors, molecules of the precursors are excited, and are then transformed into vitamin D. Therefore, the ultraviolet rays can contribute to increasing nutritive substances.

Since an LED is used as ultraviolet lamp 3118, the internal temperature of the storage case is little increased. Therefore, the ultraviolet lamp has little effect on articles stored in the storage case.

Fig. 17 is a diagram illustrating the comparison among the agricultural chemical removing performances of a method of emitting ultraviolet rays into the refrigerator according to this example, an immersion cleaning method according to the related art, a washing method using water according to the related art.

Experiments are executed as follows: an agricultural chemical removing process is performed on 10 mini tomatoes having malathion of about 3 ppm deposited thereon by using the above-mentioned three washing methods; and the concentration of malathion after the process is measured using a gas liquid chromatography to calculate a removal rate.

Each agricultural chemical removing method will be described below. 'Washing with tap water for 10 seconds' shown in Fig. 17 means that 10 mini tomatoes are put into a basket and they are washed with tap water for 10 seconds. 'Radiating ultraviolet rays for 24 hours' shown in Fig. 17 means that an ultraviolet LED that emits an ultraviolet ray having a peak wavelength of 350 nm with an intensity of 120 µW/cm2 emits ultraviolet rays to 10 mini tomatoes for 24 hours.

According to the result of the experiment, a removal rate is 20% when the tomatoes are washed with tap water for 10 seconds. That is, the result shows that 80% of the residual agricultural chemical is not removed by washing with water. When the tomatoes are washed for one hour by a washing machine using ozone water according to the related art, the removal rate of the residual agricultural chemical is 55%. In addition, when ultraviolet rays are emitted to the tomatoes for 24 hours, the removal rate of the residual agricultural chemical is 55%, which is equal to that when the washing machine is used.

As described above, since the ultraviolet LED is provided in refrigerator 3100 as ultraviolet lamp 3118, it is possible to emit ultraviolet rays to food stored in the storage case for a long time, and thus obtain the same decomposition performance as that obtained when the washing machine is used. In addition, since ultraviolet rays having a short wavelength are not used, the deterioration of the insulating wall does not occur. Therefore, the insulating wall may be formed of resin, which makes it possible to reduce manufacturing costs and lengthen the life span of the insulating wall.

In addition, since ultraviolet rays having a wavelength of about 350 nm are used, it is possible to prevent the adverse effect of ultraviolet rays on the human body, and decompose and reduce the agricultural chemicals deposited on vegetables without deteriorating the quality of vegetables.

Since the LED has a low running cost, high durability, high compatibility, and a small size the LED makes it possible to ensure a storage volume of the storage compartment.

As described above, in this example, refrigerator 3100 is provided with ultraviolet lamp 3118, and an LED that emits ultraviolet rays having a peak wavelength of about 350 nm is used as ultraviolet lamp 3118. According to this structure, it is possible to decompose agricultural chemicals, without having an adverse effect on the human body, deteriorating the quality of vegetables, and deteriorating the insulating wall formed of resin.

Further, since the LED can continuously emit ultraviolet rays, it is possible to prevent the proliferation of microorganisms deposited on stored articles and thus improve the shelf life of the stored articles.

### (Ninth example not forming part of the invention)

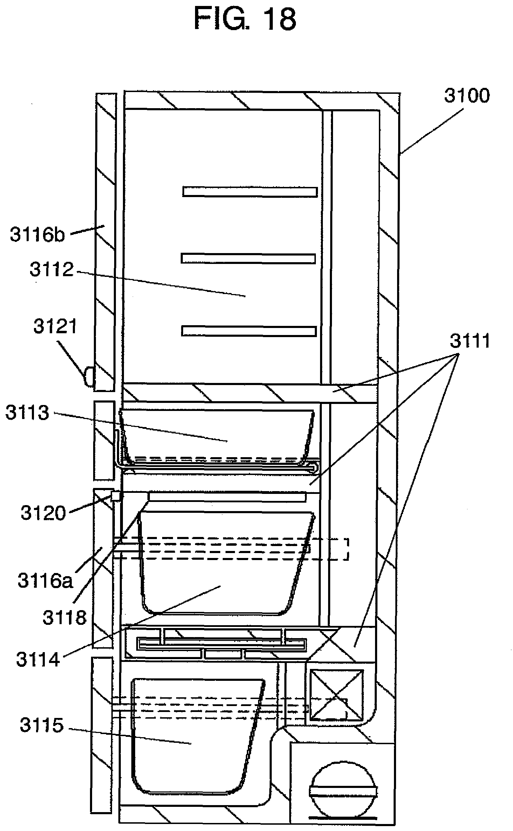

Fig. 18 is a side cross-sectional view illustrating a refrigerator according to a ninth example not forming part of the invention.

In Fig. 18, refrigerator 3100 is divided into refrigerating compartment 3112, switching chamber 3113, vegetable compartment 3114, and freezing compartment 3115 by partition plates 3111 in this order from the upper side. Door 3116a is provided in the vegetable compartment 3114 so as to close up vegetable compartment 3114, and the inner wall of vegetable compartment 3114 is formed of stainless steel. Ultraviolet lamp 3118, which is an ultraviolet emitting unit that emits ultraviolet rays having a peak wavelength of about 250 nm, is provided on the ceiling of vegetable compartment 3114. Door detector 3120 for detecting the opening or closing of door 3116a is provided in vegetable compartment 3114.

Door 3116b is provided so as to partition and close up refrigerating compartment 3112, and operating switch 3121 for turning on or off ultraviolet lamp 3118 is provided on door 3116b.

The operation and effect of the refrigerator having the above-mentioned structure will be described below.

First, the user opens door 3116a and puts vegetables into vegetable compartment 3114. Then, the user closes door 3116a. During this operation, ultraviolet lamp 3118 is in an off state. Subsequently, the user operates operating switch 3121 to turn on ultraviolet lamp 3118. Then, ultraviolet lamp 3118 emits light to articles stored in vegetable compartment 3114. Ultraviolet lamp 3118 is set such that it is automatically turned off when 30 minutes have elapsed after the switch 3121 of the ultraviolet lamp 3118 is turned on. When the user opens the door during the radiation of the ultraviolet rays, a door switch, which is door detector 3120 for detecting the opening or closing of the door, detects the opening of the door, and turns off ultraviolet lamp 3118. Therefore, light is not directly emitted to the user, and thus it is possible to ensure the safety of the user.

A lamp that emits light having a wavelength range of 220 nm to 280 nm, which has high decomposition capability, is used as ultraviolet lamp 3118. Therefore, ultraviolet rays having high photon energy are emitted to toxic substances, such as the residual agricultural chemical deposited on vegetables stored in the storage compartment, to break the chemical bond between the agricultural chemicals. In this way, it is possible to decompose the agricultural chemical components deposited on vegetables stored in the vegetable compartment, and thus reduce the agricultural chemicals deposited on vegetables stored in the vegetable compartment.

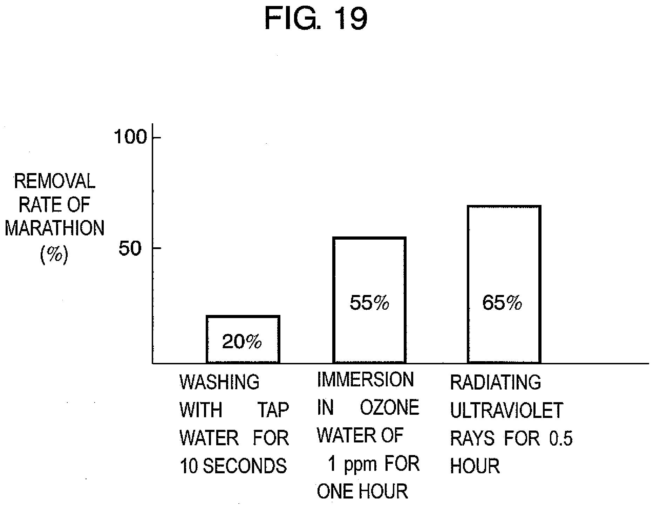

Fig. 19 is a diagram illustrating the comparison among the agricultural chemical removing performances of a method of emitting ultraviolet rays into the refrigerator according to this example, an immersion cleaning method according to the related art, a washing method using water according to the related art.

Experiments are executed as follows: an agricultural chemical removing process is performed on 10 mini tomatoes having malathion of about 3 ppm deposited thereon by using the above-mentioned three washing methods; and the concentration of malathion after the process is measured using a gas liquid chromatography to calculate a removal rate.

Each agricultural chemical removing method will be described below. 'Washing with tap water for 10 seconds' shown in Fig. 19 means that 10 mini tomatoes are put into a basket and they are washed with tap water for 10 Seconds. Radiating ultraviolet rays for 0.5 hour' shown in Fig. 19 means that an ultraviolet LED that emits an ultraviolet ray having a peak wavelength of 254 nm with an intensity of 1095 µW/cm2 emits ultraviolet rays to 10 mini tomatoes for 0.5 hour.

According to the result of the experiment, a removal rate is 20% when the tomatoes are washed with tap water for 10 seconds. That is, the result shows that 80% of the residual agricultural chemical is not removed by washing with water. When the tomatoes are washed for one hour by a washing machine using ozone water according to the related art, the removal rate of the residual agricultural chemical is 55%. In addition, when ultraviolet rays are emitted to the tomatoes for 0.5 hour, the removal rate of the residual agricultural chemical is 65%, which is higher than that when the washing machine is used. Therefore, the ultraviolet lamp 3118 is an effective unit that can decompose toxic substances, such as agricultural chemicals deposited on articles stored in the refrigerator, and thus reduce the agricultural chemicals of the stored articles, without using a dedicated device.

As described above, in this example, since ultraviolet lamp 3118 is provided in refrigerator 3100, the refrigerator can have a function of removing and decomposing toxic substances, such as agricultural chemicals, with a simple structure. Therefore, the user can simply reduce toxic substances, such as agricultural chemicals, by only storing vegetables or fruits in the refrigerator.

Further, since ultraviolet 3118 having high photon energy is used, the radiation time of ultraviolet rays can be shortened, and the deterioration of the inner wall of the vegetable compartment can be prevented.

In this example, since ultraviolet rays are emitted inside the vegetable compartment, it is possible to remove or decompose toxic substances, such as agricultural chemicals, without using water.

Since ultraviolet rays are emitted only when door detector 3120 detects the closing of door 3116a, it is possible to prevent ultraviolet rays from being emitted to the user, and thus improve the safety of the user. Since a light shielding plate is provided around the ultraviolet lamp, the emission of ultraviolet rays toward the door is blocked, and ultraviolet rays can be emitted to only the articles stored in the vegetable compartment, without being emitted to the user when the user opens the door, which results in improved safety. In addition, the light shielding plate makes it possible to emit ultraviolet rays to the articles stored in the vegetable compartment, without dispersing the energy of the ultraviolet rays emitted to the vegetable compartment. As a result, the efficiency of decomposition can be improved.

Since operating switch 3121 is provided, the user can selectively turn on or off ultraviolet lamp 3118 when detecting the operation of the ultraviolet lamp 3118. Therefore, the safety can be improved, and it is possible to decompose agricultural chemicals according to user' s needs. In addition, since the user can selectively turn on the ultraviolet lamp, if necessary, it is possible to further reduce power consumption, as compared to the case in which the ultraviolet lamp is kept in an on state at all times, which results in a reduction in the energy of the refrigerator.

In this example, the user can only turn or off operating switch 3121 for turning on or off the ultraviolet lamp. However, in addition to the on or off switching function, the ultraviolet lamp may be configured such that the user can select the amount of ultraviolet rays emitted to the vegetable compartment. In this case, the user can select the necessary amount of ultraviolet rays to be emitted, which makes it possible to selectively decompose agricultural chemicals and thus reduce power consumption.

In this example, the inner wall of the vegetable compartment is formed of stainless steel. However, the inner wall may be formed of glass or metal that is little deteriorated by ultraviolet rays. In this case, as compared to an inner wall formed of a general resin, the inner wall is less deteriorated even by ultraviolet rays having a short wavelength that is capable of effectively decomposing agricultural chemicals, or even when the amount of ultraviolet rays having the same wavelength as described above increases. Therefore, this structure can be applied to a refrigerator having an average life span of 10 years.

In this example, the light shielding plate is made of stainless steel. However, the light shielding plate may be formed of glass of metal that is little deteriorated by ultraviolet rays. In this case, the same effect as described above can be obtained.

In this example, ultraviolet lamp 3118 is provided on the ceiling of vegetable compartment 3114. However, a storage case (not shown) of vegetable compartment 3114 may be formed of a transparent material, and ultraviolet lamp 3118 may be arranged at any place inside vegetable compartment 3114. In this case, the same effect as described above can be obtained.

In this example, vegetable compartment 3114 is provided above freezing compartment 3115. However, vegetable compartment 3114 may be provided at the lowermost part. In this case, when the user uses vegetable compartment 3114, ultraviolet rays are not directly emitted to the eyes of the user, and thus the user can safely use vegetable compartment 3114.

In this example, the user turns on operating switch 3121 to emit light. However, when the door switch detects the closing of the door, the ultraviolet lamp may be turned on. When the door detector detects the opening of the door, the ultraviolet lamp may be turned off.

In this example, ultraviolet lamp 3118 is turned off when 30 minutes have elapsed after the operation of operating switch 3121. However, the user may turn off the ultraviolet lamp. In this case, the user can select the radiation time of ultraviolet rays according to user's needs. For example, the radiation time of ultraviolet rays can be determined to be long according to, for example, the amount of vegetables or vegetables with agricultural chemicals in question or according to whether the user can eat vegetables without cooking. In this way, it is possible to provide a refrigerator capable of meeting user's needs.

In this example, ultraviolet rays are emitted to the entire space of vegetable compartment 3114. However, vegetable compartment 3114 may be partitioned into a plurality of regions, and ultraviolet lamp 3118 , which is an ultraviolet ray emitting unit, may be provided in only one region. In this case, the partitioned region having ultraviolet lamp 3118 provided therein can be used as an agricultural chemical reducing region. As a result, it is possible to improve the safety of the user, and provide a refrigerator capable of meeting user's needs.

### (Tenth example not forming part of the invention)

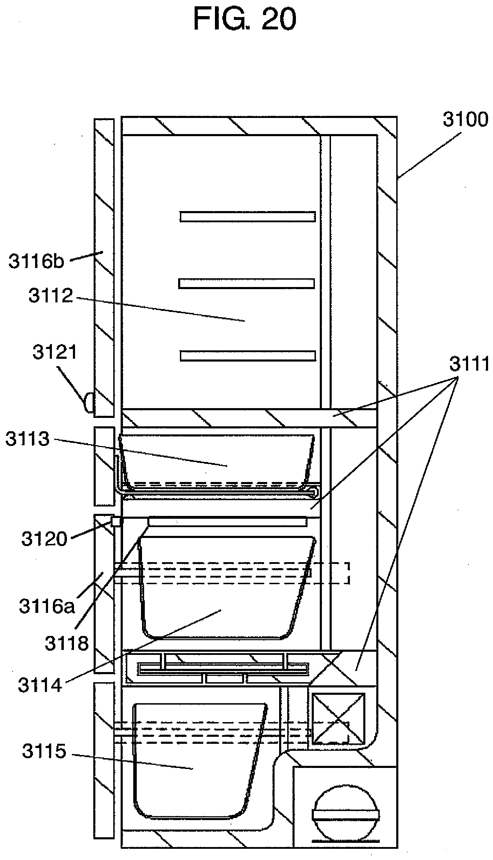

Fig. 20 is a side cross-sectional view illustrating a refrigerator according to a tenth example not forming part of the invention. In Fig. 20, the same unites and the same components as those in Fig. 16 are denoted by the same reference numerals.

In Fig. 20, refrigerator 3100 is divided into refrigerating compartment 3112, switching chamber 3113, vegetable compartment 3114, and freezing compartment 3115 by partition plates 3111 in this order from the upper side. The inner wall of vegetable compartment 3114 is formed of resin. Ultraviolet lamp 3118, serving as an ultraviolet emitting unit, is provided on the ceiling of vegetable compartment 3114. Ultraviolet lamp 3118 provided on the ceiling of vegetable compartment 3114 is an ultraviolet LED that emits ultraviolet rays having a peak wavelength of about 350 nm. An LED emitting light having a wavelength of 280 nm to 380 nm is used as the ultraviolet LED. Titanium oxide coating is performed on the entire inner surface of the vegetable compartment (not shown).

The operation and effect of the refrigerator having the above-mentioned structure will be described below.

When receiving light energy by ultraviolet rays, a titanium oxide photocatalyst can generate an OH radical having strong oxidation capacity from oxygen in the air. The OH radical can oxidize and decompose agricultural chemicals deposited on the stored articles, and thus reduce the agricultural chemical components.

In addition, the titanium oxide photocatalyst can generate ozone having high oxidation capacity from oxygen in the air. The ozone can oxidize and decompose agricultural chemicals deposited on the stored articles, and thus reduce the agricultural chemical components.

Therefore, in the refrigerator according to this example, an LED, which serves as the light source having the wavelength of the ultraviolet region, and a member including the photocatalyst that receives ultraviolet rays and serves as a catalyst are provided in the storage compartment. The member reacts with the ultraviolet rays emitted from the LED to reduce the agricultural chemical components deposited on the articles stored in the storage compartment.

In this example, a titanium oxide is coated. However, other photocatalysts may be used. In this case, the same effect as described above can be obtained. Instead of performing titanium oxide coating, the titanium oxide may be mixed with a resin material, or the photocatalyst may be formed in a cassette shape. In this case, the same effect as described above can be obtained.

In this example, as the structure of the vegetable compartment and the material forming the inner wall of the storage compartment, those described in the ninth eighth example or the ninth example may be used. Alternatively, the ultraviolet LED and the photocatalyst according to this example may be applied to the eighth example or the ninth example. In this case, the same effects as described above can be obtained.

### INDUSTRIAL APPLICABILITY

As described above, according to the refrigerators according to the above-described embodiment of the invention, a light source includes an LED emitting ultraviolet rays having a wavelength range of 280 nm to 400 nm, which is the range of relatively safe ultraviolet rays A and B, and an LED that emits visible light having a wavelength of about 400 nm or more. Light emitted from the light source makes it possible to improve the storage performance of the storage case. Therefore, the invention can be applied to apparatuses that use a relatively safe light source to improve the shelf life of articles, such as showcases, cooler boxes, and refrigerators for business.

## Claims

1. A refrigerator comprising:
a thermally insulated storage compartment (306); and
an LED (326-329) which serves as a light source (313) having a wavelength of an ultraviolet region, in the storage compartment (306),
**characterized in that** the LED includes a light transmissive cover (329), and
a portion of the cover (329) has a polarizing function.

## Patentansprüche

1. Kühlschrank, aufweisend:
ein thermisch isoliertes Aufbewahrungsfach (306); und
eine LED (326-329), die als Lichtquelle (313) dient und eine Wellenlänge in einem ultravioletten Bereich im Aufbewahrungsfach (306) aufweist,
**dadurch gekennzeichnet, dass** die LED eine lichtdurchlässige Abdeckung (329) aufweist, und ein Abschnitt der Abdeckung (329) eine polarisierende Funktion hat.

## Revendications

1. Réfrigérateur comprenant :
un compartiment de stockage thermiquement isolé (306) ; et
une diode électroluminescente (326-329) qui sert de source de lumière (313) ayant une longueur d'onde d'une région ultraviolette, dans le compartiment de stockage (306),
**caractérisé en ce que** la diode électroluminescente comprend un couvercle de transmission de lumière (329), et
une partie du couvercle (329) a une fonction de polarisation.
